(19) Europäisches Patentamt
European Patent Office
Office européen des brevets

(11) EP 0 937 459 A2

(12) # EUROPEAN PATENT APPLICATION

(43) Date of publication:
25.08.1999 Bulletin 1999/34

(51) Int. Cl.[6]: A61K 31/40

(21) Application number: 99300678.2

(22) Date of filing: 29.01.1999

(84) Designated Contracting States:
AT BE CH CY DE DK ES FI FR GB GR IE IT LI LU MC NL PT SE
Designated Extension States:
AL LT LV MK RO SI

(30) Priority: 29.01.1998 JP 3411098

(71) Applicant:
Sumitomo Pharmaceuticals Company, Limited
Osaka 541-8510 (JP)

(72) Inventors:
- Kuribayashi, Yoshikazu
Kobe-shi (JP)
- Itoh, Natsuko
Toyonaka-shi (JP)
- Ohashi, Naohito
Takatsuki-shi (JP)

(74) Representative:
Cresswell, Thomas Anthony
J.A. KEMP & CO.
14 South Square
Gray's Inn
London WC1R 5LX (GB)

(54) **Pharmaceutical compositions for the treatment of ischemic brain damage**

(57) The use of a compound which has $Na^+/H^+$ exchange system inhibition activity, in particular a substituted guanidine derivative, or a pharmaceutically acceptable acid addition salt thereof, in the manufacture of a pharmaceutical composition for the treatment of ischemic brain damage such as cerebral infarction, cerebral embolism and cerebral thrombus.

EP 0 937 459 A2

## Description

The present invention relates to a pharmaceutical composition for the treatment of ischemic brain damage. More particularly, the invention relates to a pharmaceutical composition comprising as an active ingredient a specific guanidine derivative having a $Na^+/H^+$ exchange system inhibition activity, which is particularly useful for the prevention of death at the acute phase and sequelae following ischemia, brain edema, cerebral microcirculatory disorder and for neuroprotective purpose.

The mortality rate from cerebrovascular disorders has been decreasing in these years but is still very high. Even now the number of patients with ischemic sequela or who receive treatment in hospital or attend a hospital is increasing. When ischemia in the brain continues for a certain time of period, various irreversible changes occur to cause necrosis in the brain tissue, namely, cerebral infarction, which induces various neural symptoms. Appropriate treatments during the acute phase will contribute to reduction in the mortality rate at the initial phase and to alleviation of sequelae.

$Na^+/H^+$ exchangers are plasma membrane proteins, which release $H^+$ ion outside cells concomitant with uptake of $Na^+$ ion into cells. Since the migration of $Na^+$ ion is known to be accompanied by water transfer, it is considered that $Na^+/H^+$ exchangers will regulate intracellular pH level and a cell volume. There is evidence to suggest that the $Na^+/H^+$ exchangers will be activated by ischemia and reperfusion in the tissue to increase the level of $Na^+$ ion in the cells and in turn $Ca^{2+}$ ion via $Na^+/Ca^{2+}$ exchangers, which eventually cause various cytotoxic states.

It is reported that the infarcted area in the heart caused by ischemia and reperfusion was reduced in ischemia model animals by inhibiting $Na^+/H^+$ exchangers. As far as ischemic brain damage is concerned, no report is made on any animal experiments. It is reported on a cellular level that amiloride attenuated cell swelling induced by acid loading on the cultured glial cells (Kempski, O., et al., Stroke, 19, 385-392, 1988). However, this report does not explain any correlation between brain edema and $Na^+/H^+$ exchange inhibitors, because amiloride has many other actions due to its low specificity, although it has a $Na^+/H^+$ exchange inhibition action. Rather, amiloride is used as an $Na^+/Ca^{2+}$ exchange inhibitor in some papers. Thus, the causal relation is unclear if the cell swelling was prevented by inhibiting the $Na^+/H^+$ exchange system.

Another reason to deny the reasoning that the results obtained with the cultured glial cells will explain the relationship between brain edema and $Na^+/H^+$ exchangers, is the fact that cell swelling was prevented by acetazolamide in the reported experiments. Acetazolamide is known as a diuretic agent. In fact, no significant effect of preventing cerebral edema was noted with ischemic animal models in vivo (Goto et al., NO-SOTCHU JIKKEN HANDBOOK (Experimental Handbook of Ischemic Insult), chapter 7, "Vascular permeability and brain edema", pages 629-733, published by IPC Co., Ltd.; Neurosurgery, 6, 149-154, 1980; Stroke, 2, 456-460, 1971). Reasonable reading of these reports indicates that the experimental data obtained with the cultured glial cells cannot be used as reflecting on the prevention of brain edema in vivo.

Compounds having a $Na^+/H^+$ exchange inhibitory activity are mentioned in, e.g., Japanese Patent KOKAI (Laid-Open) No. 9-67340, for use in the prevention or treatment of cerebral infarction. However, no pharmacological data was presented to support the alleged use. Indeed, there was no report to suggest that the $Na^+/H^+$ exchange inhibitory activity in association with the prevention of ischemic cerebrovascular disorders in vivo.

Main drugs currently employed for the clinical treatment of cerebral apoplexy in the acute phase are compounds like glycerol, which are considered to act through a mechanism to diminish edema by their high permeability or to prevent the blood coagulation mechanism. However, those drugs are not satisfactorily effective. A more serious problem is that anti-blood coagulants contraindicate hemorrhagic conditions or cerebral embolism. Thus, pre-tests for diagnosis become necessary, which disadvantageously retards the onset of immediate treatment required for the acute phase of ischemia. It is therefore desired to provide a drug effectively applicable to the acute phase of any conditions without requiring pre-test for diagnosis.

The present inventors have made extensive studies to develop compounds effective for the prevention of ischemic brain damage. As a result it has been found that compounds having a $Na^+/H^+$ exchange inhibitory activity exhibit an excellent effect of improving cerebral infarction and cerebral edema. Therefore the present invention has been attained. In view of the report as stated hereinabove on the experiments using the cultured glial cells in vitro, candidate compounds was elucidated in vivo in the present invention to find compounds effective for ischemic edema and infarction. It is quite likely that the $Na^+/H^+$ exchange system inhibitors will be drugs effective for the treatment of ischemic cerebrovascular disorders, especially in the acute phase, and available for the type of brain damage concomitant with hemorrhage, since $Na^+/H^+$ exchangers do not significantly affect either the blood coagulation cycle or the fibrinolytic system.

Accordingly, the present invention thus provides a pharmaceutical composition for the treatment of ischemic brain damage comprising as an active ingredient a compound having the $Na^+/H^+$ exchange system inhibition activity or a salt thereof, together with a pharmaceutically acceptable carrier.

In the preferred embodiment, said ischemic brain damage is cerebral infarction, cerebral embolism, or cerebral

thrombus.

Further, the present invention provides a method for treating ischemic brain damage, which comprises administering into a patient a compound having a $Na^+/H^+$ exchange system inhibition activity or a salt thereof in a pharmacologically effective amount.

Further, the present invention provides use of a compound having a $Na^+/H^+$ exchange system inhibition activity in the manufacture of a pharmaceutical composition for the treatment of ischemic brain damage.

Fig. 1 is a graph showing the percentage of infarcted area observed in the vehicle group and the group treated with Compound 1, which was induced in rats with transient ischemia in Example 1.
Fig. 2 is a graph showing the percentage of infarcted area observed in the vehicle group and the group treated with Compound 2, which was induced in rats with transient ischemia in Example 2.
Fig. 3 is a graph showing the percentage of infarcted area observed in the vehicle group and the group treated with Compound 3, which was induced in rats with transient ischemia in Example 3.
Fig. 4 is a graph showing the brain water content in rats with transient brain ischemia, observed in the normal control group, the vehicle group and the groups treated with 0.3 mg/kg and 1 mg/kg of Compound 1 in Example 4.
Fig. 5 is a graph showing the brain water content in rats with transient brain ischemia, observed in the normal control group, the vehicle group and the groups treated with 3 mg/kg of Compound 2 in Example 5.
Fig. 6 is a graph showing the brain water content in rats with transient brain ischemia, observed in the normal control group, the vehicle group and the groups treated with 0.3 mg/kg and 1 mg/kg of Compound 3 in Example 6.

The compounds used in the present invention as the $Na^+/H^+$ exchange system inhibitors are described below in detail.

As the $Na^+/H^+$ exchange system inhibitors or salts thereof used in the pharmaceutical compositions according to the present invention, any compounds may be used without particular restriction, so long as these compounds have an activity of inhibiting the $Na^+/H^+$ exchange system. The invention includes the following embodiments.

(a) The present invention relates to a pharmaceutical composition comprising an indoloylguanidine derivative described in Japanese Patent KOKAI (Laid-Open) No. 8-208602, which is represented by the following general formula (1):

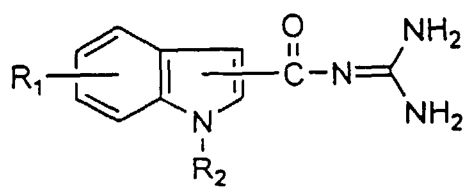

(1)

wherein:

$R_1$ represents one or more, the same or different, substituent(s) which is selected from the group consisting of a hydrogen atom, a $C_1$-$C_8$ alkyl group, a substituted $C_1$-$C_8$ alkyl group, a $C_2$-$C_6$ alkenyl group, a $C_2$-$C_6$ alkynyl group, a $C_3$-$C_7$ cycloalkyl group, a halogen atom, nitro, a $C_2$-$C_8$ alkanoyl group, an arylalkanoyl group having carbon atoms up to 10, an aroyl group having carbon atoms up to 11, carboxyl, a $C_2$-$C_6$ alkoxycarbonyl group, an aromatic group, a group shown by formula: $-OR_3$, $-NR_6R_7$, $-SO_2NR_6R_7$ or $-S(O)_nR_{40}$, and a group shown by formula:

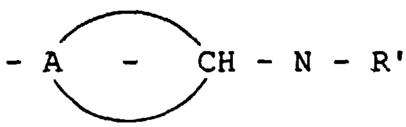

wherein A represents an oxygen atom or a group shown by formula: $-S(O)_n-$ or $-N(R_{50})-$ wherein $R_{50}$ is a hydrogen atom or a $C_1$-$C_8$ alkyl group, R' represents a hydrogen atom, a $C_1$-$C_8$ alkyl group or a substituted $C_1$-$C_8$ alkyl group; and the ring represents a saturated 3 to 8-membered hetero ring containing one nitrogen atom;

$R_2$ represents a hydrogen atom, a $C_1$-$C_8$ alkyl group, a substituted $C_1$-$C_8$ alkyl group, a $C_3$-$C_7$ cycloalkyl group, hydroxy, a $C_1$-$C_6$ alkoxy group, an aromatic group or a group shown by formula: $-CH_2R_{20}$;

$R_3$ represents a hydrogen atom, a $C_1$-$C_8$ alkyl group, a substituted $C_1$-$C_8$ alkyl group, a $C_3$-$C_7$ cycloalkyl group, an aromatic group or a group shown by formula: $-CH_2R_{30}$, in which $R_{30}$ represents an $C_2$-$C_6$ alkenyl group or an $C_2$-$C_6$ alkynyl group;

each of $R_6$ and $R_7$ independently represents a hydrogen atom, a $C_1$-$C_8$ alkyl group, a substituted $C_1$-$C_8$ alkyl group, a $C_3$-$C_7$ cycloalkyl group, a $C_2$-$C_8$ alkanoyl group, an arylalkanoyl group having carbon atoms up to 10, an aroyl group having carbon atoms up to 11, an aromatic group or a group shown by formula: $-CH_2R_{60}$ wherein $R_{60}$ represents a $C_2$-$C_6$ alkenyl group or a $C_2$-$C_6$ alkynyl group; or $R_6$ and $R_7$ are combined together to form a saturated 5- to 7-membered cyclic amino group which may contain other hetero atom(s) in the ring thereof;

$R_{40}$ represents a $C_1$-$C_8$ alkyl group, a substituted $C_1$-$C_8$ alkyl group or an aromatic group;

n represents 0, 1 or 2; and,

$R_{20}$ represents a $C_2$-$C_6$ alkenyl group or a $C_2$-$C_6$ alkynyl group;

in which:

the substituent (s) of the substituted $C_1$-$C_8$ alkyl group means a halogen atom, hydroxy, a $C_1$-$C_6$ alkoxy group, cyano, carboxyl, a $C_2$-$C_6$ alkoxycarbonyl group, a $C_2$-$C_8$ alkanoyl group, an arylalkanoyl group having carbon atoms up to 10, an aroyl group having carbon atoms up to 11, an aromatic group, and $-CONR_4R_5$ in which each of $R_4$ and $R_5$ independently represents a hydrogen atom or a $C_1$-$C_8$ alkyl group or $R_4$ and $R_5$ are combined together to form a saturated 5- to 7-membered cyclic amino group which may contain other hetero atom(s) in the ring; $-NR_6R_7$; or a group shown by:

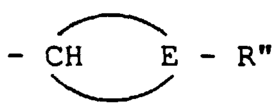

in which:

E represents a nitrogen atom or a CH group and

R" represents a hydrogen atom, a $C_1$-$C_8$ alkyl group or a substituted $C_1$-$C_8$ alkyl group substituted with hydroxy, a $C_1$-$C_6$ alkoxy group, cyano, carboxyl, a $C_2$-$C_6$ alkoxycarbonyl group, a $C_2$-$C_8$ alkanoyl group, an arylalkanoyl group having carbon atoms up to 10, an aroyl group having carbon atoms up to 11, an aromatic group, a group shown by $-NR_6R_7$, or a group shown by $-CONR_4R_5$, in which each of $R_4$ and $R_5$ independently represents a hydrogen atom or a $C_1$-$C_8$ alkyl group or $R_4$ and $R_5$ are combined together to form a saturated 5- to 7-membered cyclic amino group which may contain other hetero atom(s) therein; and the ring of

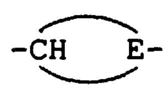

is a 3-to 8-membered saturated aliphatic ring or saturated hetero ring containing one nitrogen atom;

all of the aromatic groups hereinabove means an aryl group having carbon atoms up to 10, a 5- or 6-membered heteroaryl group containing 1 to 4 nitrogen atom(s), a 5- or 6-membered hetero-aryl group containing 1 to 2 nitrogen atom(s) and one oxygen atom or one sulfur atom, or furyl; and,

all of the aromatic groups hereinabove may be substituted with a substituent selected from the group consisting of a $C_1$-$C_8$ alkyl group, a substituted $C_1$-$C_8$ alkyl group, a halogen atom, nitro, a $C_2$-$C_6$ alkoxycarbonyl group, carboxyl and a group selected from the group shown by formulae: $-OR_3$, $-NR_6R_7$, $-CONR_6R_7$, $-SO_2NR_6R_7$ and $-S(O)_nR_{40}$;

provided that $R_1$ and the guanidinocarbonyl group may be substituted at any one of the 5- and 6-membered rings of the indole nucleus;

or,

a pharmaceutically acceptable acid addition salt thereof.

The respective groups in the indoloylguanidine derivatives of the general formula (1) are described below in detail.

The alkyl group refers to a linear or branched alkyl group having 8 or less carbon atoms, for example, methyl, ethyl, propyl, 2-propyl, butyl, 2-butyl, 2-methylpropyl, 1,1-dimethylethyl, pentyl, hexyl, heptyl and octyl.

The alkenyl group includes, for example, alkenyl groups of 6 or less carbon atoms, such as vinyl, allyl, propenyl, 2-propenyl, butenyl, pentenyl and hexenyl.

The alkynyl group includes, for example, alkynyl groups of 6 or less carbon atoms, such as ethynyl, propargyl, butynyl and pentynyl.

The cycloalkyl group refers to a cycloalkyl group having 3 to 7 carbon atoms, e.g., cyclopropyl, cyclobutyl, cyclopentyl, cyclohexyl and cycloheptyl.

Typical examples of the halogen atom include fluorine, chlorine and bromine.

The acyl group refers to a linear or branched alkanoyl group having carbon atoms up to 8, e.g., acetyl, propanoyl and 2-methylpropanoyl; an arylalkanoyl group having carbon atoms up to 10, e.g., phenylacetyl and phenylpropanoyl; and an aroyl group having 11 or less carbon atoms, e.g., benzoyl, 1-naphthoyl and 2-naphthoyl.

The alkoxycarbonyl group refers to a linear or branched alkoxycarbonyl group having carbon atoms up to 6, e.g., methoxycarbonyl, ethoxycarbonyl, propoxycarbonyl and 2-propoxycarbonyl.

The aromatic group refers to an aryl or hetero-aryl group which may have a substituent. Examples of the aryl group are those having carbon atoms up to 10, e.g., phenyl, tolyl or naphthyl, and examples of the hetero-aryl group are a 5- or 6-membered aromatic group containing 1 to 4 nitrogen atoms or a 5- or 6-membered aromatic ring containing 1 to 2 nitrogen atoms and one oxygen atom or one sulfur atom, e.g., 2-, 3- or 4-pyridyl, imidazolyl, triazolyl, tetrazolyl, 2- or 3-furyl, 2- or 3-thienyl, 1-, 3- or 4-oxazolyl, and 3-, 4-or 5-isoxazolyl.

Examples of the substituent in the substituted aryl or hetero-aryl group include an alkyl group, a substituted alkyl group, a halogen atom, nitro, an alkoxycarbonyl group, carboxyl and a group shown by formula: $-0R_3$, $-NR_6R_7$, $-CONR_6R_7$, $-SO_2NR_6R_7$ or $-S(O)_nR_{40}$.

Where $R_1$ is a group shown by formula: $-OR_3$ wherein $R_3$ is an aromatic group, representative examples of the $-OR_3$ group include phenoxy and a substituted phenoxy group. Examples of the substituted phenoxy group are a phenoxy group substituted with nitro, $-NR_6R_7$ wherein $R_6$ and $R_7$ are typically a hydrogen atom or an alkyl group, or a substituted alkyl group, the substituent of which is exemplified by hydroxy or $-NR_6R_7$. Specific examples of the substituted phenoxy group are o-, m- or p-nitrophenoxy, o-, m- or p-aminophenoxy, o-, m- or p-(dimethylamino)phenoxy, o-, m- or p-(aminomethyl)phenoxy and o-, m- or p-(dimethylaminomethyl)phenoxy.

The alkoxy group refers to a linear or branched alkoxy group having carbon atoms up to 6, e.g., methoxy, ethoxy, isopropoxy and tert-butoxy.

As the saturated 5- to 7-membered cyclic amino group which is formed by combining $R_6$ and $R_7$ together and may contain other hetero atoms therein, there are, for example, a 5- to 7-membered cyclic group containing 1 to 3 nitrogen atoms and a 5- to 7-membered cyclic group containing one nitrogen atom and one oxygen atom. Specific examples of such cyclic amino group include 1-pyrrolidinyl, piperidino, 1-piperazinyl, morpholino, 4-methylmorpholino and 4-methylpiperazinyl.

Examples of the substituent in the substituted alkyl group include a cycloalkyl group, a halogen atom, hydroxy, an alkoxy group, cyano, carboxyl, an alkoxycarbonyl group, an acyl group, an aromatic group, or a group shown by formula: $-CONR_4R_5$, wherein each of $R_4$ and $R_5$ independently represents hydrogen atom or an alkyl group, or $R_4$ and $R_5$ are combined together to form a saturated 5- to 7-membered cyclic amino group which may contain other hetero atoms in the ring; $-NR_6R_7$; or a group shown by formula:

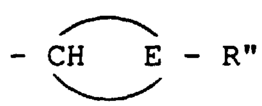

wherein:

E represents a nitrogen atom or a CH group and
R" represents a hydrogen atom, an alkyl group or a substituted alkyl group substituted with hydroxy, a $C_1$-$C_6$ alkoxy group, cyano, carboxyl, a $C_2$-$C_6$ alkoxycarbonyl group, a $C_2$-$C_8$ alkanoyl group, an arylalkanoyl group having carbon atoms up to 10, an aroyl group having carbon atoms up to 11, an aromatic group, a group shown by $-NR_6R_7$, or a group shown by $-CONR_4R_5$, in which each of $R_4$ and $R_5$ independently represents a hydrogen atom or a $C_1$-$C_8$ alkyl group or $R_4$ and $R_5$ are combined together to form a saturated 5- to 7-membered cyclic amino group which may contain other hetero atom(s) therein; and the ring of

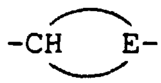

is a 3- to 8-membered saturated aliphatic ring or saturated hetero ring containing one nitrogen atom. Particularly where $R_1$, $R_2$ and $R_3$ represent a substituted alkyl group, examples of the substituent include a cycloalkyl group, a halogen atom, hydroxy, an alkoxy group, carboxyl, an alkoxycarbonyl group, an acyl group, an aromatic group or a group shown by formula: $-CONR_4R_5$ or $-NR_6R_7$. Where $R_6$ and $R_7$ represent a substituted alkyl group, examples of the substituent include a cycloalkyl group, hydroxy, an alkoxy group, carboxyl, an alkoxycarbonyl group, an acyl group, an aryl group or a group shown by formula: $-CONR_4R_5$ or $-NR_4R_5$. As the alkyl moiety in the substituted alkyl group, the same examples as those for the alkyl group described above are given.

As such a substituted alkyl group, there are, for example, an alkyl group having 1 to 5 carbon atoms which is substituted with a cycloalkyl having 3 to 6 carbon atoms, a polyhaloalkyl group having 1 to 5 carbon atoms, a hydroxyalkyl group having 1 to 6 carbon atoms, an alkoxyalkyl group having 2 to 6 carbon atoms, a cyanoalkyl group having 2 to 6 carbon atoms, a carboxyalkyl group having 2 to 6 carbon atoms, an alkoxycarbonylalkyl group having 3 to 8 carbon atoms, an alkanoylalkyl group having 3 to 8 carbon atoms, an aralkyl group having carbon atoms up to 16, a phenyl- or naphthyl-$C_1$ to $C_5$ alkyl group which may be substituted, a carbamoyl-$C_1$ to $C_3$ alkyl group in which the nitrogen atom may be substituted with one or two $C_1$ to $C_3$ alkyl, an amino-$C_1$ to $C_5$ alkyl group in which the nitrogen atom may be substituted with one or two $C_1$ to $C_3$ alkyl or $C_7$ to $C_{11}$ aralkyl, and a saturated 5- to 7-membered cyclic amino-$C_1$ to $C_3$ alkyl group.

Representative examples of the substituted alkyl group include:

in the case of $R_1$: a polyhaloalkyl group having 1 to 3 carbon atoms such as trifluoromethyl, trifluoroethyl or trichloromethyl; a hydroxyalkyl group having 1 to 6 carbon atoms such as hydroxymethyl, hydroxyethyl or 1-hydroxyethyl; and an aminoalkyl group having 1 to 5 carbon atoms such as aminomethyl, aminoethyl or 1-aminoethyl;

in the case of $R_2$: a hydroxyalkyl group having 1 to 6 carbon atoms such as hydroxyethyl, hydroxypropyl, hydroxybutyl, 2-hydroxypropyl or 3,4-dihydroxybutyl; an alkoxyalkyl group having 1 to 6 carbon atoms such as methoxyethyl, ethoxyethyl or methoxypropyl; a carboxyalkyl group having 2 to 6 carbon atoms such as carboxyethyl or carboxypropyl; an alkoxycarbonylalkyl group having 3 to 7 carbon atoms such as methoxycarbonylmethyl, ethoxycarbonylmethyl or methoxycarbonylethyl; a phenyl- or naphthyl-$C_1$ to $C_5$ alkyl group, wherein a phenyl or naphthyl group may be substituted with a substituent, e.g., a $C_1$ to $c_3$ alkyl group, a halogen atom, nitro, amino, hydroxy or a $C_1$ to $C_3$ alkoxy group, such as benzyl, phenylethyl, phenylpropyl, phenylbutyl or, 1- or 2-naphthylmethyl; a carbamoyl-$C_1$ to $c_3$ alkyl group in which the nitrogen atom may be substituted with one or two $C_1$ to $C_3$ alkyl groups, such as carbamoylmethyl, carbamoylethyl or dimethylcarbamoylmethyl; or, an amino-$C_1$ to $C_5$ alkyl group in which the nitrogen atom may be substituted with one or two $C_1$ to $C_3$ alkyl, such as aminoethyl, aminopropyl, dimethylaminoethyl, dimethylaminopropyl or diethylaminoethyl;

in the case of $R_3$ and $R_{40}$: a hydroxyalkyl group having 1 to 6 carbon atoms such as hydroxyethyl, hydroxypropyl, 2-hydroxypropyl, hydroxybutyl or 2,3-dihydroxybutyl; a carboxyalkyl group having 2 to 6 carbon atoms such as carboxymethyl or carboxyethyl; a phenyl-$C_1$ to $C_5$ alkyl group such as benzyl, phenylethyl or phenylpropyl; a carbamoyl-$C_1$ to $C_3$ alkyl group such as carbamoylmethyl or carbamoylethyl; an amino-$C_1$ to $C_5$ alkyl group containing one or two nitrogen atoms in which the nitrogen atom may be substituted with one or two $C_1$ to $C_3$ alkyl or $C_7$ to $C_{11}$ aralkyl groups, such as aminoethyl, aminopropyl, dimethylaminoethyl, dimethylaminopropyl or benzylmethyl-aminoethyl; or a saturated 5- to 7-membered cyclic amino-$C_1$ to $C_3$ alkyl group such as 1-pyrrolidinyl-ethyl or piperidinoethyl; and,

in the case of $R_6$ and $R_7$: a phenyl-$C_1$ to $C_5$ alkyl group such as phenylethyl.

Examples of the saturated 5- to 7-membered cyclic amino group which is formed by combining $R_4$ and $R_5$ together and may contain other hetero atoms in the ring thereof include the same groups as exemplified for the aforesaid cyclic amino group formed by $R_6$ and $R_7$.

Examples of the group shown by formula: $-S(O)_nR_{40}$ include an alkylsulfonyl group having carbon atoms up to 8, such as methylsulfonyl, ethylsulfonyl, propylsulfonyl or isopropylsulfonyl; and the corresponding alkylsulfinyl and alkylthio groups.

Examples of the group of include the following groups:

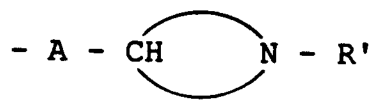

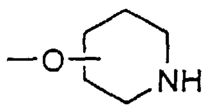 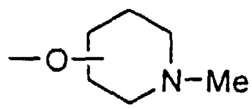 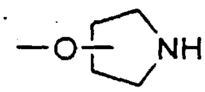 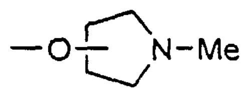

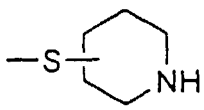 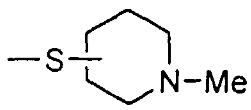 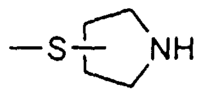 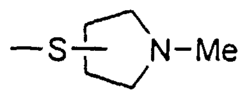

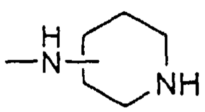 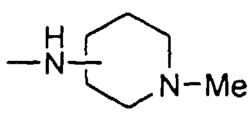 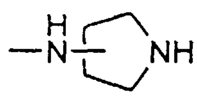 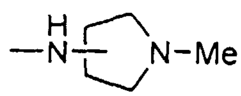

Among the above groups, there are preferred (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-methylpiperidin-4-yl)thio, (pyrrolidin-3-yl)thio, (1-methylpyrrolidin-3-yl)thio, (piperidin-3-yl)amino, (piperidin-4-yl)amino, (1-methylpiperidin-3-yl)amino, (1-methylpiperidin-4-yl)amino, (pyrrolidin-3-yl)amino and (1-methylpyrrolidin-3-yl)amino.

(b) The present invention relates to a pharmaceutical composition comprising a substituted guanidine derivative described in EP 787,728, which is represented by the general formula (1b):

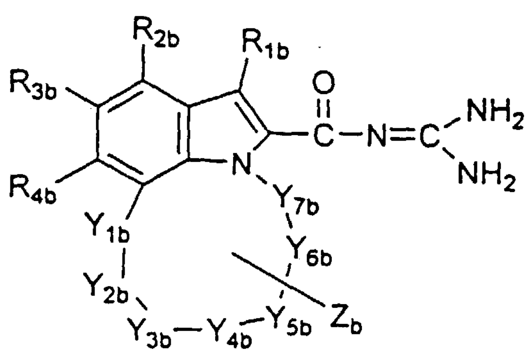

(1b)

wherein:

$R_{1b}$, $R_{2b}$, $R_{3b}$ and $R_{4b}$ are independently a hydrogen atom, an unsubstituted alkyl group, a substituted alkyl group, a cycloalkyl group, a cycloalkenyl group, a saturated heterocyclic group, a halogen atom, a nitro group, a carboxyl group, an alkoxycarbonyl group, an aromatic group, an acyl group, $-OR_{5b}$, $-N(R_{6b})R_{7b}$, $-CON(R_{6b})R_{7b}$, $-SO_2N(R_{6b})R_{7b}$, $-S(O)_nR_{8b}$ wherein $R_{8b}$ is an unsubstituted alkyl group, a substituted alkyl group or an aromatic group, and n is 0, 1 or 2, $-Q_b-R_{ab}$, or

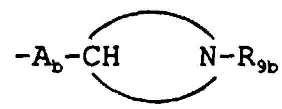

wherein $A_b$ is an oxygen atom or $-S(O)_n-$ wherein n is as defined above or $-N(R_{10b})-$, $R_{9b}$ is a hydrogen atom, an unsubstituted alkyl group, a substituted alkyl group, an acyl group or $-Q_b-R_{ab}$, and the ring is a 3- to 8-membered saturated heterocyclic group composed of a nitrogen atom and carbon atoms;

$Y_{1b}$, $Y_{2b}$, $Y_{3b}$, $Y_{4b}$, $Y_{5b}$, $Y_{6b}$ and $Y_{7b}$, which may be the same or different, are independently a single bond, $-CH_2-$, -O-, -CO-, $-C(=C(R_{11b})R_{12b})-$, $-S(O)_n-$ or $-N(R_{10b})-$, adjacent members of a group consisting of $Y_{1b}$ through $Y_{7b}$ being able to be taken together to represent -CH=CH-, and at least two of $Y_{1b}$ through $Y_{7b}$ being independently a group other than a single bond;

$Z_b$ may be absent, or one or more $Z_b$s may be present and are, the same or different, independently the following substituent for a hydrogen atom bonded to any of the carbon atoms constituting the ring formed by $Y_{1b}$ through $Y_{7b}$; an unsubstituted alkyl group, a substituted alkyl group, an alkenyl group, an alkynyl group, a cycloalkyl group, a cycloalkenyl group, a substituted heterocyclic group, a halogen atom, a carboxyl group, an alkoxycarbonyl group, an aromatic group, an acyl group, $-OR_{5b}$, $-N(R_{6b})R_{7b}$, $-S(O)_nR_{8b}$, $-C(O)N(R_{6b})R_{7b}$, or $-Q_b-R_{ab}$, provided that when $Z_b$ is a substituent for the hydrogen atom of -CH=CH-, $Z_b$ is not $-N(R_{6b})R_{7b}$ or $-S(O)_nR_{8b}$;

$Q_b$ is a substituted or unsubstituted lower alkylene group;

$R_{ab}$ is a substituted or unsubstituted vinyl group, or a substituted or unsubstituted ethynyl group;

$R_{5b}$ is a hydrogen atom, an unsubstituted alkyl group, a substituted alkyl group, a cycloalkyl group, a cycloalkenyl group, a saturated heterocyclic group or an aromatic group;

$R_{6b}$ and $R_{7b}$ are independently a hydrogen atom, an unsubstituted alkyl group, a substituted alkyl group, a cycloalkyl group, a cycloalkenyl group, a saturated heterocyclic group, an aromatic group, an acyl group or $-Q_b-R_{ab}$, or $R_{6b}$ and $R_{7b}$, when taken together with the nitrogen atom to which they are bonded, form a saturated 5- to 7-membered cyclic amino group which may contain an oxygen atom or a sulfur atom in the ring and may be substituted by one or more unsubstituted alkyl groups, substituted alkyl groups, hydroxyl groups or $-OR_{5b}$ groups;

$R_{8b}$ is an unsubstituted alkyl group, a substituted alkyl group or an aromatic group;

$R_{10b}$ is a hydrogen atom, an unsubstituted alkyl group, a substituted alkyl group, a cycloalkyl group, a saturated heterocyclic group, an aromatic group, an acyl group or $-Q_b-R_{ab}$; and,

$R_{11b}$ and $R_{12b}$ are independently a hydrogen atom, an unsubstituted alkyl group, a substituted alkyl group, an alkenyl group, an alkynyl group, a cycloalkyl group, a cycloalkenyl group, a saturated heterocyclic group, a halogen atom, a carboxyl group, an alkoxycarbonyl group, an aromatic group, an acyl group, $-OR_{5b}$, $-N(R_{6b})R_{7b}$, $-C(O)N(R_{6b})R_{7b}$, $-S(O)_nR_{8b}$ or $-Q_bR_{ab}$; and n is 0, 1 or 2;

or a pharmaceutically acceptable acid addition salt thereof.

The various groups in the compounds of general formula (1b) are explained below.

The alkyl group includes, for example, linear or branched alkyl groups of 8 or less carbon atoms, such as methyl, ethyl, propyl, 2-propyl, butyl, 2-butyl, 2-methylpropyl, 1,1-dimethylethyl, pentyl, hexyl, heptyl and octyl.

The cycloalkyl group may be unsubstituted or may be substituted by 1 to 4 unsubstituted alkyl groups, substituted alkyl groups, hydroxyl groups or groups of the formula $-OR_{5b}$, and includes, for example, 3- to 8-membered cycloalkyl groups such as cyclopropyl, cyclobutyl, cyclopentyl, cyclohexyl, cycloheptyl, 2-methylcyclopentyl, 3-methylcyclopentyl, 2-methylcyclohexyl, 3-methylcyclohexyl, 4-methylcyclohexyl, 2-hydroxycyclopentyl, 3-hydroxycyclopentyl, 2-hydroxycyclohexyl, 3-hydroxycyclohexyl, 4-hydroxycyclohexyl, 2-(hydroxymethyl)cyclopentyl, 3-(hydroxymethyl)cyclopentyl, 2-(hydroxymethyl)cyclohexyl, 3-(hydroxymethyl)-cyclohexyl, 4-(hydroxymethyl)cyclohexyl, 2-(aminomethyl)cyclopentyl, 3-(aminomethyl)cyclopentyl, 2-(aminomethyl)cyclohexyl, 3-(aminomethyl)cyclohexyl, 4-(aminomethyl)cyclohexyl, 2-(methoxymethyl)cyclopentyl, 3-(methoxymethyl)cyclopentyl, 2-(methoxymethyl)cyclohexyl, 3-(methoxymethyl)cyclohexyl, 4-(methoxymethyl)-cyclohexyl, etc.

The cycloalkenyl group may be unsubstituted or may be substituted by 1 to 4 unsubstituted alkyl groups, substituted alkyl groups, hydroxyl groups or groups of the formula, $-OR_{5b}$, and includes, for example, 3- to 8-membered cycloalkenyl groups having a double bond, such as 1-cyclopentenyl, 2-cyclopentenyl, 3-cyclopentenyl 1-cyclohexenyl, 2-cyclohexenyl, 3-cyclohexenyl, etc.

The saturated heterocyclic group may be unsubstituted or may be substituted by 1 to 4 unsubstituted alkyl

groups, substituted alkyl groups, hydroxyl groups or group of the formula $-OR_{5b}$, and includes, for example, 3- to 8-membered saturated heterocyclic groups having an oxygen atom or a sulfur atom, such as 2-tetrahydrofuranyl, 3-tetrahydrofuranyl, 2-tetrahydro-2H-pyranyl, 4-tetrahydro-4H-pyranyl, etc.

The halogen atom includes, for example, fluorine, chlorine and bromine atoms.

The alkoxycarbonyl group includes, for example, linear or branched alkoxycarbonyl group of 6 or less carbon atoms, such as methoxycarbonyl, ethoxycarbonyl, propoxycarbonyl and 2-propoxycarbonyl.

The aromatic group includes substituted or unsubstituted aryl groups and substituted or unsubstituted heteroaryl groups. As the aryl groups, there may be exemplified aryl groups of 10 or less carbon atoms, such as phenyl, naphthyl, etc. As the heteroaryl groups, there may be exemplified 5- or 6-membered heteroaryl groups containing 1 to 4 nitrogen atoms, such as 2-, 3- or 4-pyridyl, imidazolyl, triazolyl, tetrazolyl, etc.; and 5- or 6-membered heteroaryl groups containing 0 to 2 nitrogen atoms and one oxygen atom or one sulfur atom, such as 2- or 3-furyl, 2- or 3-thienyl, 1-, 3- or 4-oxazolyl, 3-, 4- or 5-isoxazolyl, etc.

The substituent of each of the substituted aryl group and the substituted heteroaryl group includes unsubstituted alkyl groups, substituted alkyl groups, halogen atoms, nitro group, carboxyl groups, alkoxycarbonyl groups, and groups represented by the formula $-OR_{5b}$, $-N(R_{6b})R_{7b}$, $-CON(R_{6b})R_{7b}$, $-SO_2N(R_{6b})R_{7b}$ or $-S(O)_nR_{8b}$.

When $R_{1b}$, $R_{2b}$, $R_{3b}$ and $R_{4b}$ are independently a group represented by the formula $-OR_{5b}$ wherein $R_{5b}$ is an aromatic group, typical examples of the $-OR_{5b}$ group are unsubstituted phenoxy groups and substituted phenoxy groups. Examples of the substituted phenoxy groups are those having as the substituent, for example, a nitro group, a $-N(R_{6b})R_{7b}$ group wherein $R_{6b}$ and $R_{7b}$ are independently, for instance, a hydrogen atom or an unsubstituted alkyl group, or a substituted alkyl group having as the substituent, for example, a hydroxyl group or a $-N(R_{6b})R_{7b}$ group. More specific examples of the substituted phenoxy group are o-, m- or p-nitrophenoxy, o-, m- or p-aminophenoxy, o-, m- or p-(dimethylamino)-phenoxy, o-, m- or p-(aminomethyl)phenoxy, and o-, m- or p-(dimethylaminomethyl)phenoxy.

The alkoxy group includes, for example, linear or branched alkoxy groups of 6 or less carbon atoms, such as methoxy, ethoxy, isopropoxy and tert-butoxy.

As the cyclic amino group which $R_{6b}$ and $R_{7b}$ form when taken together with the nitrogen atom to which they are bonded, i.e., the saturated 5- to 7-membered cyclic amino group which may contain another hetero atom in the ring, there may be exemplified 5- to 7-membered cyclic groups containing 1 to 3 nitrogen atoms and 5- to 7-membered cyclic groups containing one nitrogen atom and one oxygen atom. More specific examples of the saturated 5- to 7-membered cyclic amino group are 1-pyrrolidinyl, 1-piperidino, 1-piperazinyl, morpholino and 1-(4-methyl)piperazinyl.

The substituent on the substituted alkyl group include halogen atoms, hydroxy group, alkoxy groups, cycloalkyl groups, cyano group, carboxyl group, alkoxycarbonyl groups, acyl groups, aromatic groups, and groups shown by the formula $-CONR_{pb}R_{qb}$ (wherein $R_{pb}$ and $R_{qb}$ are independently a hydrogen atom or an unsubstituted alkyl group, or $R_{pb}$ and $R_{qb}$ being able to be taken together to represent a saturated 5- to 7-membered cyclic amino group which may contain another hetero atom in the ring), $-NR_{6b}R_{7b}$ or a group shown by formula:

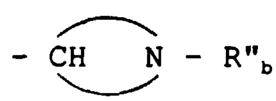

wherein R" is a hydrogen atom, an unsubstituted alkyl group or a substituted alkyl group, and the ring is a 3-to 8-membered saturated heterocyclic group containing one nitrogen atom. Particularly when $R_{1b}$, $R_{2b}$, $R_{3b}$, $R_{4b}$, $R_{5b}$, $R_{8b}$, $R_{11b}$, $R_{12b}$, or $Z_b$ is a substituted alkyl group, the substituent includes, for example, cycloalkyl groups, halogen atoms, hydroxyl group, alkoxy groups, carboxyl group, alkoxycarbonyl groups, acyl groups, aromatic groups and groups shown by formula $-CONR_{pb}R_{qb}$ or $-NR_{6b}R_{7b}$. When $R_{6b}$, $R_{7b}$, $R_{9b}$ or $R_{10b}$ is a substituted alkyl group, the substituent includes, for example, cycloalkyl groups, hydroxyl group, alkoxy groups, carboxyl group, alkoxycarbonyl groups, acyl groups, aryl groups and groups shown by the formula $-CONR_{pb}R_{qb}$ or $-NR_{pb}R_{qb}$. As the alkyl portion of the substituted alkyl group, there may be exemplified the same groups as those exemplified above as the alkyl group.

As such substituted alkyl groups, there may be exemplified substituted alkyl groups of 1 to 5 carbon atoms having as the substituent a cycloalkyl group of 3 to 6 carbon atoms, polyhaloalkyl groups of 1 to 5 carbon atoms, hydroxyalkyl groups of 1 to 6 carbon atoms, alkoxyalkyl groups of 2 to 6 carbon atoms, cyanoalkyl groups of 2 to 6 carbon atoms, carboxyalkyl groups of 2 to 6 carbon atoms, alkoxycarbonylalkyl groups of 3 to 8 carbon atoms, alkanoylalkyl groups of 3 to 8 carbon atoms, aroylalkyl groups of 16 or less carbon atoms, substituted or unsubstituted phenyl- or naphthyl-$C_1$-$C_5$ alkyl groups, carbamoyl-$C_1$-$C_3$ alkyl groups which may have one or two $C_1$-$C_3$ alkyl

groups as a substituent(s) on the nitrogen atom, amino-$C_1$-$C_5$ alkyl groups which may have one or two $C_1$-$C_3$ alkyl or $C_7$-$C_{11}$ aralkyl groups as a substituent(s) on the nitrogen atom, and saturated 5- to 7-membered cyclic amino-$C_1$-$C_3$ alkyl groups.

Typical examples of the substituted alkyl group are polyhaloalkyl groups of 1 to 3 carbon atoms, such as trifluoromethyl, trifluoroethyl or trichloromethyl; hydroxyalkyl groups of 1 to 6 carbon atoms, such as hydroxymethyl, hydroxyethyl or 1-hydroxyethyl; aminoalkyl groups of 1 to 5 carbon atoms, such as aminomethyl, aminoethyl or 1-aminoethyl; alkoxyalkyl groups of 1 to 6 carbon atoms, such as methoxyethyl, ethoxyethyl or methoxypropyl; carboxyalkyl groups of 2 to 6 carbon atoms, such as carboxyethyl or carboxypropyl; alkoxycarbonylalkyl groups of 3 to 7 carbon atoms, such as methoxycarbonylmethyl, ethoxycarbonylmethyl or methoxycarbonylethyl; phenyl- or naphthyl-$C_1$-$C_5$ alkyl groups (which may have in the phenyl or naphthyl portion a substituent such as a $C_1$-$C_3$ alkyl group, halogen atom, nitro group, amino group, hydroxyl group, $C_1$-$C_3$ alkoxy group or the like) such as benzyl, phenylethyl, phenylpropyl, phenylbutyl or, 1- or 2-naphthylmethyl; carbamoyl-$C_1$-$C_3$ alkyl groups which may have one or two $C_1$-$C_3$ alkyl groups as a substituent(s) on the nitrogen atom, for example, carbamoylmethyl, carbamoylethyl or dimethylcarbamoyl-methyl; amino-$C_1$-$C_5$ alkyl groups which may have one or two $C_1$-$C_3$ alkyl groups or $C_7$-$C_{11}$ aralkyl groups as a substituent(s) on the nitrogen atom, for example, aminoethyl, aminopropyl, dimethylaminoethyl, dimethylaminopropyl, diethylaminoethyl or N-methyl-N-benzylaminoethyl; and saturated 5- to 7-membered cyclic amino-$C_1$-$C_3$ alkyl groups such as 1-pyrrolidinyl-ethyl or piperidinoethyl. In the case of $R_{6b}$ and $R_{7b}$, typical examples of the substituted alkyl group are phenyl-$C_1$-$C_5$ alkyl groups such as phenylethyl.

As the substituent on the lower alkylene group for $Q_b$ and the substituent on the vinyl or ethynyl group for $R_{ab}$, there may be exemplified unsubstituted alkyl groups, substituted alkyl groups, cycloalkyl groups, cycloalkenyl groups, saturated heterocyclic groups, carboxyl group, alkoxycarbonyl groups, aromatic groups, and groups represented by the formula -CON($R_{6b}$)$R_{7b}$.

The lower alkylene group includes, for example, alkylene groups of 6 or less carbon atoms, such as methylene, ethylene, trimethylene, tetramethylene, pentamethylene, hexamethylene, etc.

The acyl group includes, for example, formyl group; alkanoyl groups of 2 to 6 carbon atoms, such as acetyl, propanoyl, etc.; cycloalkanecarbonyl groups of 3 to 6 carbon atoms, such as cyclopropanecarbonyl, cyclobutanecarbonyl, cyclopentanecarbonyl, cyclohexanecarbonyl, etc.; cycloalkenecarbonyl groups of 3 to 6 carbon atoms, such as cyclopentenecarbonyl, cyclohexenecarbonyl, etc.; aroyl groups of 6 to 10 carbon atoms, such as benzoyl, toluoyl, naphthoyl, etc.; saturated heterocyclic ring-carbonyl groups having a 5- or 6-membered saturated heterocyclic group containing one or two hetero atoms selected from the group consisting of nitrogen atom, oxygen atom and sulfur atom, for example, 2-piperidinecarbonyl, 3-morpholinecarbonyl, etc.; and heteroaryl acyl groups having a 5- or 6-membered heteroaryl ring containing one or two hetero atoms selected from the group consisting of nitrogen atom, oxygen atom and sulfur atom, for example, furoyl, thenoyl, nicotinoyl, isonicotinoyl, etc.

As the cyclic amino group which $R_{pb}$ and $R_{bq}$ form when taken together, i.e., the saturated 5- to 7-membered cyclic amino group which may contain another hetero atom in the ring, there may be exemplified the same groups as those exemplified above as the cyclic amino group formed by $R_{6b}$ and $R_{7b}$.

The group represented by the formula -S(O)$_n$$R_{8b}$ includes, for example, alkylsulfonyl groups of 8 or less carbon atoms, such as methylsulfonyl, ethylsulfonyl, propylsulfonyl or isopropylsulfonyl; and the corresponding alkylsulfinyl groups and alkylthio groups.

As the group represented by the formula:

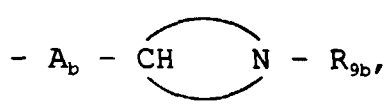

there may be exemplified groups represented by the following formulas:

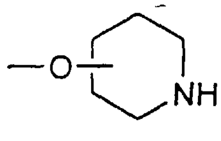 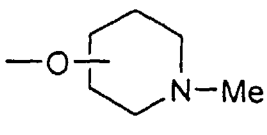 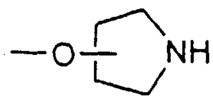 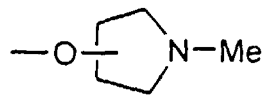

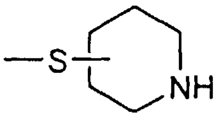 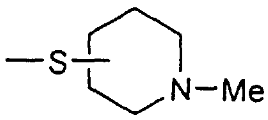 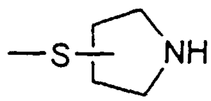 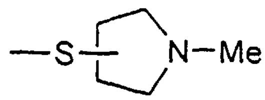

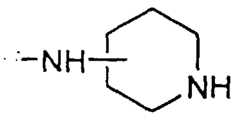 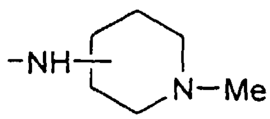 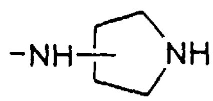 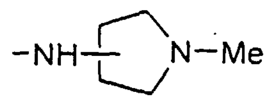

Preferred examples of the aforesaid group are (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-methylpiperidin-4-yl)thio, (pyrrolidin-3-yl)thio, (1-methylpyrrolidin-3-yl)thio, (piperidin-3-yl)amino, (piperidin-4-yl)amino, (1-methylpiperidin-3-yl)amino, (1-methylpiperidin-4-yl)amino, (pyrrolidin-3-yl)amino and (1-methylpyrrolidin-3-yl)amino.

The alkenyl group includes, for example, alkenyl groups of 6 or less carbon atoms, such as vinyl, allyl, propenyl, 2-propenyl, butenyl, pentenyl and hexenyl.

The alkynyl group includes, for example, alkynyl groups of 6 or less carbon atoms, such as ethynyl, propargyl, butynyl and pentynyl.

As $Y_{1b}$, $Y_{2b}$, $Y_{3b}$, $Y_{4b}$, $Y_{5b}$, $Y_{6b}$ and $Y_{7b}$, the following may be exemplified.

1. One of $Y_{1b}$ through $Y_{7b}$ is $-CH_2-$, -O-, -CO-, $-C(=C(R_{11b})R_{12b})-$, $-S(O)_n-$ or $-N(R_{10b})-$, another is $-CH_2-$, and the five others, which may be the same or different, are independently a single bond or $-CH_2-$. More specific examples of $Y_{1b}$ through $Y_{7b}$ are as follows.

1-1. $Y_{1b}$ is $-CH_2-$, -O-, -CO-, $-C(=C(R_{11b})R_{12b})-$, $-S(O)_n-$ or $-N(R_{10b})-$, $Y_{2b}$ is $-CH_2-$, and $Y_{3b}$ through $Y_{7b}$, which may be the same or different, are independently a single bond or $-CH_2-$.
1-2. $Y_{7b}$ is -O-, -CO-, $-C(=C(R_{11b})R_{12b})-$, $Y_{6b}$ is $-CH_2-$, and $Y_{1b}$ through $Y_{5b}$, which may be the same or different, are independently a single bond or $-CH_2-$.
1-3. $Y_{1b}$ and $Y_{7b}$ are $-CH_2-$, one of $Y_{2b}$, $Y_{3b}$, $Y_{4b}$, $Y_{5b}$ and $Y_{6b}$ is $-CH_2-$, -O-, $-C(=C(R_{11b})R_{12b})-$, $-S(O)_n-$ or $-N(R_{10b})-$, and the four others, which may be the same or different, are independently a single bond or $-CH_2-$.
1-4. $Y_{1b}$ is $-CH_2-$, -O-, -CO-, $-C(=C(R_{11b})R_{12b})-$, $-S(O)_n-$ or $-N(R_{10b})-$, $Y_{2b}$ through $Y_{4b}$ are independently $-CH_2-$, and $Y_{5b}$ and $Y_{6b}$ are independently a single bond.

2. Any adjacent two members of a group consisting of $Y_{1b}$ through $Y_{7b}$ are taken together to represent -CH=CH-, the four others, which may be the same or different, are independently a single bond or $-CH_2-$, and $Y_{7b}$ is a single bond, -O-, -CO-, $-C(=C(R_{11b})R_{12b})-$or $-CH_2-$.

More specific examples of $Y_{1b}$ through $Y_{7b}$ are as follows.

2-1. $-Y_{1b}-Y_{2b}-$ is -CH=CH-.
2-2. $Y_{1b}$ is $-CH_2-$, and $-Y_{2b}-Y_{3b}-$ is -CH=CH-.
2-3. $Y_{1b}$ and $Y_{2b}$ are independently $-CH_2-$, and $-Y_{3b}-Y_{4b}-$ is -CH=CH-.
2-4. $Y_{1b}$, $Y_{2b}$ and $Y_{3b}$ are $-CH_2-$, and $-Y_{4b}-Y_{5b}-$ is -CH=CH-.

3. $Y_{1b}$ is -O- or $-N(R_{10b})-$, one of $Y_{2b}$ through $Y_{7b}$ is -CO-, and the five others, which may be the same or different, are independently a single bond or $-CH_2-$.

More specific examples of $Y_{1b}$ through $Y_{7b}$ are as follows.

3-1. $Y_{2b}$ is -CO-.
3-2. $Y_{2b}$ is $-CH_2-$, and $Y_{3b}$ is -CO-.
3-3. $Y_{2b}$ and $Y_{3b}$ are $-CH_2-$, and $Y_{4b}$ is -CO-.
3-4. $Y_{2b}$, $Y_{3b}$ and $Y_{4b}$ are $-CH_2-$, and $Y_{5b}$ is -CO-.
3-5. $Y_{2b}$, $Y_{3b}$, $Y_{4b}$ and $Y_{5b}$ are $-CH_2-$, and $Y_{6b}$ is - CO-.

Preferred examples of $Y_{1b}$ through $Y_{7b}$ are such that two of five, in particular, two to four, of $Y_{1b}$ through $Y_{7b}$ are independently a single bond and the others independently a group other than a single bond. More preferably, two or three of $Y_{1b}$ through $Y_{7b}$ are independently a single bond, and the others are groups other than a single bond.

(c) The present invention relates to a pharmaceutical composition comprising a substituted guanidine derivative described in Japanese Patent KOKAI (Laid-Open) No. 9-291076, which is represented by the general formula (1c):

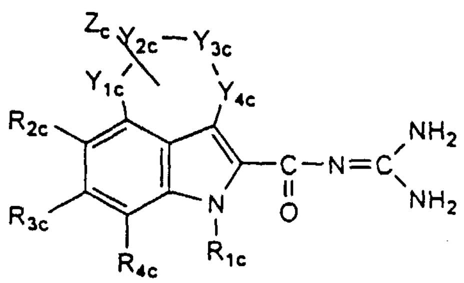

(1c)

wherein

$R_{1c}$ is a hydrogen atom, an unsubstituted alkyl group, a substituted alkyl group, a cycloalkyl group, a cycloalkenyl group, a saturated heterocyclic group, an aromatic group, $-OR_{5c}$, an acyl group or $-Q_c-R_{ac}$;
$R_{2c}$, $R_{3c}$ and $R_{4c}$ are independently a hydrogen atom, an unsubstituted alkyl group, a substituted alkyl group, a cycloalkyl group, a cycloalkenyl group, a saturated heterocyclic group, a halogen atom, a nitro group, a carboxyl group, an alkoxycarbonyl group, an aromatic group, $-OR_{5c}$, $-N(R_{6c})R_{7c}$, $-CON(R_{6c})R_{7c}$, $-SO_2N(R_{6c})R_{7c}$, $-S(O)_nR_{8c}$, an acyl group, $-Q_c-R_{ac}$, or

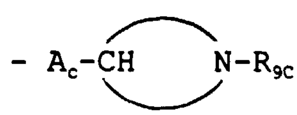

wherein the ring is a 3- to 8-membered saturated heterocyclic group composed of a nitrogen atom and carbon atoms;
$Y_{1c}$, $Y_{2c}$, $Y_{3c}$ and $Y_{4c}$ are as follows:

(1) one of $Y_{1c}$, $Y_{2c}$, $Y_{3c}$ and $Y_{4c}$ is a methylene group, a carbonyl group, an oxygen atom, $-S(O)_n-$, $-N(R_{10c})-$ or $-C(=C(R_{11c})(R_{12c}))-$, two others are independently a methylene group, and the remaining one is a single bond or a methylene group, or,
(2) any adjacent two members of a group consisting of $Y_{1c}$, $Y_{2c}$, $Y_{3c}$, and $Y_{4c}$ are taken together to represent a vinylene group (-CH=CH-) or $-CON(R_{10c})-$, another is a methylene group, a carbonyl group, an oxygen atom, $-S(O)_n-$, $-N(R_{10c})$ - or $-C(=C(R_{11c})(R_{12c}))-$, and the remaining one is a single bond or a methylene group, provided that the oxygen atom, nitrogen atom and sulfur atom are not directly bonded to the vinylene group;

$Z_c$ is a substituent which may be substituted for at least one hydrogen atom (for example, one or two hydrogen atoms) bonded to any of the carbon atoms constituting the ring formed by $Y_{1c}$, $Y_{2c}$, $Y_{3c}$, and $Y_{4c}$, namely, $Z_c$ may be absent, or one or more Zs may be present and are independently a substituent selected from the group consisting of unsubstituted alkyl groups, substituted alkyl groups, alkenyl

groups, alkynyl groups, cycloalkyl groups, cycloalkenyl groups, saturated heterocyclic groups, halogen atoms, carboxyl groups, alkoxycarbonyl groups, aromatic groups, $-OR_{5c}$, $-N(R_{6c})R_{7c}$, $-CON(R_{6c})R_{7c}$, $-S(O)_nR_{8c}$, acyl groups and $-Q_c-R_{ac}-$;

$A_c$ is an oxygen atom, $-S(O)_n-$ or $-N(R_{10c})-$;

$Q_c$ is a substituted or unsubstituted lower alkylene group;

$R_{ac}$ is a substituted or unsubstituted vinyl group, or a substituted or unsubstituted ethynyl group;

$R_{5c}$ is a hydrogen atom, an unsubstituted alkyl group, a substituted alkyl group, a cycloalkyl group, a cycloalkenyl group, a saturated heterocyclic group or an aromatic group;

$R_{6c}$ and $R_{7c}$ are independently a hydrogen atom, an unsubstituted alkyl group, a substituted alkyl group, a cycloalkyl group, a cycloalkenyl group, a saturated heterocyclic group, an aromatic group, an acyl group or $-Q_c-R_{ac}$, or $R_{6c}$ and $R_{7c}$, when taken together with the nitrogen atom to which they are bonded, form a saturated 5- to 7-membered cyclic amino group which may contain an oxygen atom or a sulfur atom in the ring and may be substituted by one or more (for example, two) unsubstituted alkyl groups, substituted alkyl groups, hydroxyl groups or $-OR_{5c}$ groups;

$R_{8c}$ is an unsubstituted alkyl group, a substituted alkyl group or an aromatic group;

$R_{9c}$ is a hydrogen atom, an unsubstituted alkyl group, a substituted alkyl group, an acyl group or $-Q_c-R_{ac}$,

$R_{10c}$ is a hydrogen atom, an unsubstituted alkyl group, a substituted alkyl group, a cycloalkyl group, a saturated heterocyclic group, an aromatic group, an acyl group or $-Q_c-R_{ac}$; and,

$R_{11c}$ and $R_{12c}$ are independently a hydrogen atom, an unsubstituted alkyl group, a substituted alkyl group, an alkenyl group, an alkynyl group, a cycloalkyl group, a cycloalkenyl group, a saturated heterocyclic group, a halogen atom, a carboxyl group, an alkoxycarbonyl group, an aromatic group, $-OR_{5c}$, $-N(R_{6c})R_{7c}$, $-CON(R_{6c})R_{7c}$, $-S(O)_nR_{8c}$, an acyl group or $-Q_c-R_{ac}-$; and,

n is 0, 1 or 2; or a pharmaceutically acceptable acid addition salt thereof.

The various groups in the compounds of general formula (1c) are explained below.

The alkyl group includes, for example, linear or branched alkyl groups of 8 or less carbon atoms, such as methyl, ethyl, propyl, 2-propyl, butyl, 2-butyl, 2-methylpropyl, 1,1-dimethylethyl, pentyl, hexyl, heptyl and octyl.

The cycloalkyl group may be unsubstituted or may be substituted by 1 to 4 unsubstituted alkyl groups, substituted alkyl groups, hydroxyl groups or groups of the formula $-OR_{5c}$, and includes, for example, 3- to 8-membered cycloalkyl groups such as cyclopropyl, cyclobutyl, cyclopentyl, cyclohexyl, cycloheptyl, 2-methylcyclopentyl, 3-methylcyclopentyl, 2-methylcyclohexyl, 3-methylcyclohexyl, 4-methylcyclohexyl, 2-hydroxycyclopentyl, 3-hydroxycyclopentyl, 2-hydroxycyclohexyl, 3-hydroxycyclohexyl, 4-hydroxycyclohexyl, 2-(hydroxymethyl)cyclopentyl, 3-(hydroxymethyl)cyclopentyl, 2-(hydroxymethyl)cyclohexyl, 3-(hydroxymethyl)-cyclohexyl, 4-(hydroxymethyl)cyclohexyl, 2-(aminomethyl)cyclopentyl, 3-(aminomethyl)cyclopentyl, 2-(aminomethyl)cyclohexyl, 3-(aminomethyl)cyclohexyl, 4-(aminomethyl)cyclohexyl, 2-(methoxymethyl)cyclopentyl, 3-(methoxymethyl)cyclopentyl, 2-(methoxymethyl)cyclohexyl, 3-(methoxymethyl)cyclohexyl, 4-(methoxymethyl)-cyclohexyl, etc.

The cycloalkenyl group may be unsubstituted or may be substituted by 1 to 4 unsubstituted alkyl groups, substituted alkyl groups, hydroxyl groups or groups of the formula, $-OR_{5c}$, and includes, for example, 3- to 8-membered cycloalkenyl groups having a double bond, such as 1-cyclopentenyl, 2-cyclopentenyl, 3-cyclopentenyl 1-cyclohexenyl, 2-cyclohexenyl, 3-cyclohexenyl, etc.

The saturated heterocyclic group may be unsubstituted or may be substituted by 1 to 4 unsubstituted alkyl groups, substituted alkyl groups, hydroxyl groups or group of the formula $-OR_{5c}$, and includes, for example, 3- to 8-membered saturated heterocyclic groups having an oxygen atom or a sulfur atom, such as 2-tetrahydrofuranyl, 3-tetrahydrofuranyl, 2-tetrahydro-2H-pyranyl, 4-tetrahydro-4H-pyranyl, etc.

The halogen atom includes, for example, fluorine, chlorine and bromine atoms.

The alkoxycarbonyl group includes, for example, linear or branched alkoxycarbonyl group of 6 or less carbon atoms, such as methoxycarbonyl, ethoxycarbonyl, propoxycarbonyl and 2-propoxycarbonyl.

The aromatic group includes substituted or unsubstituted aryl groups and substituted or unsubstituted heteroaryl groups. As the aryl groups, there may be exemplified aryl groups of 10 or less carbon atoms, such as phenyl, naphthyl, etc. As the heteroaryl groups, there may be exemplified 5- or 6-membered heteroaryl groups containing 1 to 4 nitrogen atoms, such as 2-, 3- or 4-pyridyl, pyrrolyl, imidazolyl, triazolyl, tetrazolyl, etc.; and 5- or 6-membered heteroaryl groups containing 0 to 2 nitrogen atoms and one oxygen atom or one sulfur atom, such as 2- or 3-furyl, 2- or 3-thienyl, 1-, 3- or 4-oxazolyl, 3-, 4- or 5-isoxazolyl, etc. The substituent of each of the substituted

aryl group and the substituted heteroaryl group includes unsubstituted alkyl groups, substituted alkyl groups, halogen atoms, nitro group, carboxyl groups, alkoxycarbonyl groups, and groups represented by the formula $-OR_{5c}$, $-N(R_{6c})R_{7c}$, $-CONR_{6c}R_{7c}$, $-SO_2NR_{6c}R_{7c}$ or $-S(O)_nR_{8c}$.

When $R_{1c}$, $R_{2c}$, $R_{3c}$ and $R_{4c}$ are independently a group represented by the formula $-OR_{5c}$ wherein $R_{5c}$ is an aromatic group, typical examples of the $-OR_{5c}$ group are unsubstituted phenoxy groups and substituted phenoxy groups. Examples of the unsubstituted phenoxy groups are those having as the substituent, for example, a nitro group, a $-NR_{6c}R_{7c}$ group (wherein $R_{6c}$ and $R_{7c}$ are independently, for instance, a hydrogen atom or an unsubstituted alkyl group), or a substituted alkyl group having as the substituent, for example, a hydroxyl group or a $-NR_{6c}R_{7c}$ group. More specific examples of the substituted phenoxy groups are o-, m- or p-nitrophenoxy, o-, m- or p-aminophenoxy, o-, m- or p-(dimethylamino)phenoxy, o-, m- or p-(aminomethyl)phenoxy, and o-, m- or p-(dimethylaminomethyl)phenoxy.

The alkoxy group includes, for example, linear or branched alkoxy groups of 6 or less carbon atoms, such as methoxy, ethoxy, isopropoxy, tert-butoxy, etc.

As the cyclic amino group which $R_{6c}$ and $R_{7c}$ form when taken together with the nitrogen atom to which they are bonded, i.e., the saturated 5- to 7-membered cyclic amino group which may contain another hetero atom in the ring, there may be exemplified 5- to 7-membered cyclic groups containing 1 to 3 nitrogen atoms and 5- to 7-membered cyclic groups containing one nitrogen atom and one oxygen atom. More specific examples of the saturated 5- to 7-membered cyclic amino group are 1-pyrrolidinyl, 1-piperidino, 1-piperazinyl, 4-morpholino and 1-(4-methyl)piperazinyl.

The substituent on the substituted alkyl group include halogen atoms, hydroxy group, alkoxy groups, cycloalkyl groups, cyano group, carboxyl group, alkoxycarbonyl groups, aromatic groups, acyl groups and groups shown by the formula $-CONR_{pc}R_{qc}$, wherein $R_{pc}$ and $R_{qc}$ are independently a hydrogen atom or an unsubstituted alkyl group, or $R_{pc}$ and $R_{qc}$ being able to be taken together to represent a saturated 5- to 7-membered cyclic amino group which may contain another hetero atom in the ring, $-NR_{6c}R_{7c}$ or a group shown by formula:

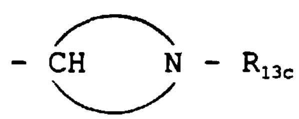

wherein $R_{13c}$ is a hydrogen atom, an unsubstituted alkyl group or a substituted alkyl group, and the ring is a 3-to 8-membered saturated heterocyclic group containing one nitrogen atom. Particularly when any of $R_{1c}$, $R_{2c}$, $R_{3c}$, $R_{4c}$, $R_{5c}$, $R_{8c}$, $R_{9c}$, $R_{12c}$ and $Z_c$ is a substituted alkyl group, the substituent includes, for example, cycloalkyl groups, halogen atoms, hydroxyl group, alkoxy groups, carboxyl group, alkoxycarbonyl groups, acyl groups, aromatic groups and groups shown by the formula $-CONR_{pc}R_{qc}$ or $-NR_{6c}R_{7c}$. When any of $R_{6c}$, $R_{7c}$, $R_{10c}$, $R_{11c}$ and $R_{13c}$ is a substituted alkyl group, the substituent includes, for example, cycloalkyl groups, hydroxyl group, alkoxy groups, carboxyl group, alkoxycarbonyl groups, acyl groups, aryl groups, and groups shown by the formula $-CONR_{pc}R_{qc}$ or $-NR_{pc}R_{qc}$. As the alkyl portion of the substituted alkyl group, there may be exemplified the same groups as those exemplified above as the alkyl group.

As such substituted alkyl groups, there may be exemplified substituted alkyl groups of 1 to 5 carbon atoms having as the substituent a cycloalkyl group of 3 to 6 carbon atoms, polyhaloalkyl groups of 1 to 5 carbon atoms, hydroxyalkyl groups of 1 to 6 carbon atoms, alkoxyalkyl groups of 2 to 6 carbon atoms, cyanoalkyl groups of 2 to 6 carbon atoms, carboxyalkyl groups of 2 to 6 carbon atoms, alkoxycarbonylalkyl groups of 3 to 8 carbon atoms, alkanoylalkyl groups of 3 to 8 carbon atoms, aroylalkyl groups of 16 or less carbon atoms, substituted or unsubstituted phenyl- or naphthyl-$C_1$-$C_5$ alkyl groups, carbamoyl-$C_1$-$C_3$ alkyl groups which may have one or two $C_1$-$C_3$ alkyl groups as a substituent(s) on the nitrogen atom, amino-$C_1$-$C_5$ alkyl groups which may have one or two $C_1$-$C_3$ alkyl or $C_7$-$C_{11}$ aralkyl groups as a substituent(s) on the nitrogen atom, and saturated 5- to 7-membered cyclic amino-$C_1$-$C_3$ alkyl groups.

Typical examples of the substituted alkyl group are polyhaloalkyl groups of 1 to 3 carbon atoms, such as trifluoromethyl, trifluoroethyl or trichloromethyl; hydroxyalkyl groups of 1 to 6 carbon atoms, such as hydroxymethyl, hydroxyethyl or 1-hydroxyethyl; aminoalkyl groups of 1 to 5 carbon atoms, such as aminomethyl, aminoethyl or 1-aminoethyl; alkoxyalkyl groups of 1 to 6 carbon atoms, such as methoxyethyl, ethoxyethyl or methoxypropyl; carboxyalkyl groups of 2 to 6 carbon atoms, such as carboxyethyl or carboxypropyl; alkoxycarbonylalkyl groups of 3 to 7 carbon atoms, such as methoxycarbonylmethyl, ethoxycarbonylmethyl or methoxycarbonylethyl; phenyl- or naphthyl-$C_1$-$C_5$ alkyl groups (which may have in the phenyl or naphthyl portion a substituent such as a $C_1$-$C_3$ alkyl group, halogen atom, nitro group, amino group, hydroxyl group, $C_1$-$C_3$ alkoxy group or the like) such as benzyl, phenylethyl, phenylpropyl, phenylbutyl or, 1- or 2-naphthylmethyl; carbamoyl-$C_1$-$C_3$ alkyl groups which may have one or two $C_1$-$C_3$ alkyl groups as a substituent(s) on the nitrogen atom, for example, carbamoylmethyl, carbamoylethyl or dimethylcarbamoylmethyl; amino-$C_1$-$C_5$ alkyl groups which may have one or two $C_1$-$C_3$ alkyl groups or $C_7$-$C_{11}$ aralkyl groups as a substituent(s) on the nitrogen atom, for example, aminoethyl, aminopropyl, dimethylaminoethyl,

dimethylaminopropyl, diethylaminoethyl or N-methyl-N-benzylaminoethyl; and saturated 5-to 7-membered cyclic amino-$C_1$-$C_3$ alkyl groups such as 1-pyrrolidinylethyl or piperidinoethyl. In the case of $R_{6c}$ and $R_{7c}$, typical examples of the substituted alkyl group are phenyl-$C_1$-$C_5$ alkyl groups such as phenylethyl.

As the substituent on the lower alkylene group for $Q_c$ and the substituent on the vinyl or ethynyl group for $R_{ac}$, there may be exemplified unsubstituted alkyl groups, substituted alkyl groups, cycloalkyl groups, cycloalkenyl groups, saturated heterocyclic groups, carboxyl group, alkoxycarbonyl groups, aromatic groups, and groups represented by the formula $-CON(R_{6c})R_{7c}$.

The lower alkylene group includes, for example, alkylene groups of 6 or less carbon atoms, such as methylene, ethylene, trimethylene, tetramethylene, pentamethylene, hexamethylene, etc.

The acyl group includes, for example, formyl group; alkanoyl groups of 2 to 6 carbon atoms, such as acetyl, propanoyl, etc.; cycloalkanecarbonyl groups of 3 to 6 carbon atoms, such as cyclopropanecarbonyl, cyclobutanecarbonyl, cyclopentanecarbonyl, cyclohexanecarbonyl, etc.; cycloalkenecarbonyl groups of 3 to 6 carbon atoms, such as cyclopentenecarbonyl, cyclohexenecarbonyl, etc.; aroyl groups of 6 to 10 carbon atoms, such as benzoyl, toluoyl, naphthoyl, etc.; saturated heterocyclic ring-carbonyl groups having a 5- or 6-membered saturated heterocyclic group containing one or two hetero atoms selected from the group consisting of nitrogen atom, oxygen atom and sulfur atom, for example, 2-piperidinecarbonyl, 3-morpholinecarbonyl, etc.; and heteroaromatic acyl groups having a 5- or 6-membered heteroaromatic ring containing one or two hetero atoms selected from the group consisting of nitrogen atom, oxygen atom and sulfur atom, for example, furoyl, thenoyl, nicotinoyl, isonicotinoyl, etc.

As the cyclic amino group which $R_{pc}$ and $R_{qc}$ form when taken together, i.e., the saturated 5- to 7-membered cyclic amino group which may contain another hetero atom in the ring, there may be exemplified the same groups as those exemplified above as the cyclic amino group formed by $R_{6c}$ and $R_{7c}$.

The group represented by the formula $-S(O)_nR_{8c}$ includes, for example, alkylsulfonyl groups of 8 or less carbon atoms, such as methylsulfonyl, ethylsulfonyl, propylsulfonyl or isopropylsulfonyl; and alkylsulfinyl groups and alkylthio groups, which correspond to the alkylsulfonyl groups.

As the group represented by the formula:

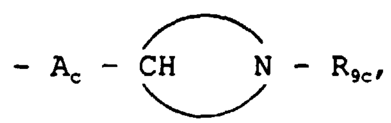

there may be exemplified groups represented by the following formulas:

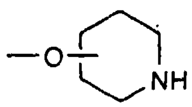, 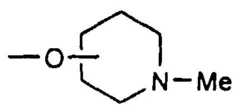, 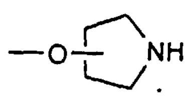, 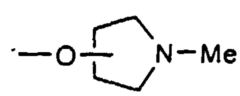,

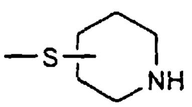, 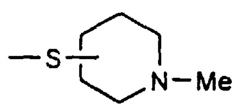, 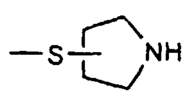, 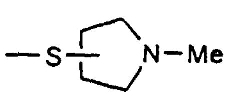,

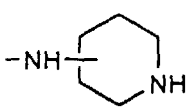, 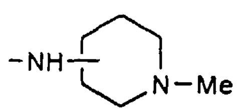, 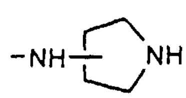, 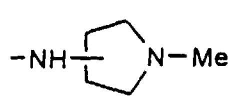

Preferred examples of the aforesaid group are (piperidin-3-yl)oxy, (piperidin-4-yl)oxy, (1-methylpiperidin-3-yl)oxy, (1-methylpiperidin-4-yl)oxy, (pyrrolidin-3-yl)oxy, (1-methylpyrrolidin-3-yl)oxy, (piperidin-3-yl)thio, (piperidin-4-yl)thio, (1-methylpiperidin-3-yl)thio, (1-methylpiperidin-4-yl)thio, (pyrrolidin-3-yl)thio, (1-methylpyrrolidin-2-yl)thio, (piperidin-3-yl)amino, (piperidin-4-yl)amino, (1-methylpiperidin-3-yl)amino, (1-methylpiperidin-4-yl)amino, (pyrrolidin-3-yl)amino and (1-methylpyrrolidin-3-yl)amino.

The alkenyl group includes, for example, alkenyl groups of 6 or less carbon atoms, such as vinyl, allyl, propenyl, 2-propenyl, butenyl, pentenyl and hexenyl.

The alkynyl group includes, for example, alkynyl groups of 6 or less carbon atoms, such as ethynyl, propargyl, butynyl and pentynyl.

(d) The present invention relates to pharmaceutical compositions comprising Compound (d) represented by formula (1d):

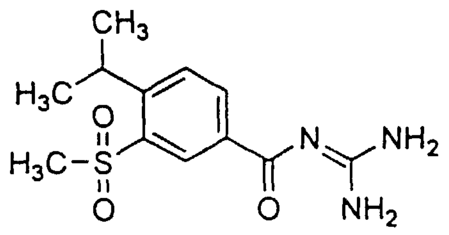

(1d)

or pharmaceutically acceptable acid addition salts thereof, in particular, the methanesulfonate (Code No. HOE-642, cariporide, namely, 4-isopropyl-3-methylsulfonylbenzoylguanidine methanesulfonate).

(e) The present invention relates to pharmaceutical compositions comprising Compound (e) represented by formula (1e):

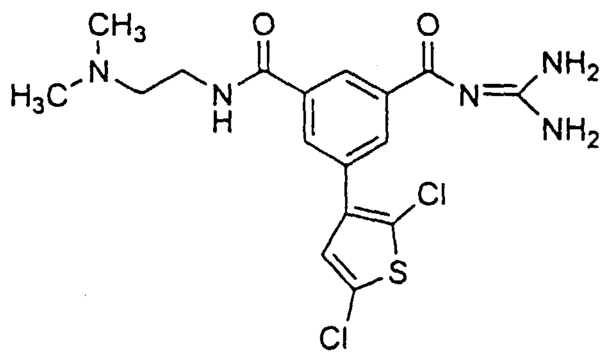

(1e)

or pharmaceutically acceptable acid addition salts thereof, in particular, the dihydrochloride (Code No. FR183998).

(f) The present invention relates to pharmaceutical compositions comprising Compound (f) represented by formula (1f):

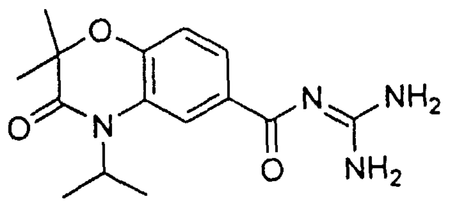

(1f)

or pharmaceutically acceptable acid addition salts thereof, in particular, the methanesulfonate (Code No. KBR-9032).

Of the compounds described above, particularly preferred are Compounds (g) and (h) shown below.

(g) 1-Methyl-2-indoloylguanidine represented by the formula (1g) described in Japanese Patent KOKAI (Laid-Open) No. 8-208602:

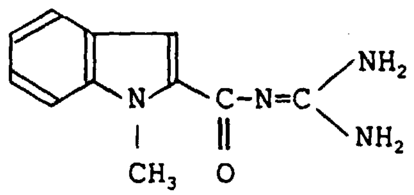

(1g)

or salts thereof, more preferably the methanesulfonate thereof.

(h) N-(Aminoiminomethyl)-5,6,7,8-tetrahydro11-chloro-8-oxo-4H-azocino[3,2,1-hi]indole-2-carboxamide of the formula (1h) described in EP 787,728:

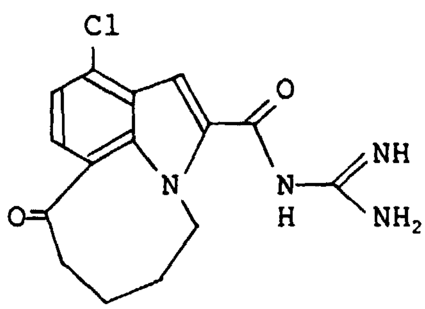

(1h)

or salt thereof, more preferably, the methanesulfonate thereof.

Throughout the present specification, the compounds represented by the general formulas (1), (1b) and (1c) and the compounds of the formulas (1d), (1e), (1f), (1g) and (1h) are described using the formulas (1), (1b), (1c), (1d), (1e), (1f), (1g) and (1h) but tautomers of these compounds are also present since the guanidine portions are tautomeric. More specifically, the tautomer of acylguanidine group: $-C(O)N=C(NH_2)_2$ in which the guanidine portion takes diaminomethyleneamino as well as that of $-C(O)NH-C(=NH)NH_2$ in which the guanidine portion takes aminoiminomethylamino are present in the respective compounds. These tautomeric compounds are different only in their chemical formation state but have the same pharmacological effects. Therefore, the compounds which may be used in the pharmaceutical compositions of the present invention include these tautomeric isomers.

The compounds of the general formulas (1), (1b) and (1c) also include those having optically asymmetric centers. Thus, these compounds may be obtained in the racemic form, or can be obtained in the optically active form in the case that optically active starting materials are employed. If necessary and desired, the racemic compounds thus obtained may resolved chemically or physically into enantiomers by known methods. Preferably, the racemic compounds are subjected to a reaction using an optically active resolving agent to convert into the diastereomers. The diastereomers in different configurations may be resolved in a conventional manner, for example, by fractional crystallization.

The compounds represented by the general formulas (1), (1b) and (1c) and the compounds represented by the formulas (1d), (1e), (1f), (1g) and (1h) may be converted into acid addition salts with pharmaceutically acceptable inorganic acids or organic acids, if necessary and desired. Examples of such acid addition salts are salts with inorganic acids such as hydrochloric acid, hydrobromic acid, sulfuric acid or phosphoric acid; salts with organic acids such as formic acid, acetic acid, fumaric acid, maleic acid, oxalic acid, citric acid, malic acid, tartaric acid, aspartic acid or glutamic acid; salts with sulfonic acids such as methanesulfonic acid, benzenesulfonic acid, p-toluenesulfonic acid, hydroxybenzenesulfonic acid, dihydroxybenzenesulfonic acid, etc.

The compounds represented by the general formulas (1), (1b) and (1c) and the compounds represented by the formulas (1d), (1e), (1f), (1g) and (1h), and acid addition salts thereof may take the form of the anhydrides, hydrates and solvates thereof.

As the $Na^+/H^+$ exchanger inhibitors which can be used in the pharmaceutical compositions of the present invention, compounds described in a publication, such as the one described in the following publications may also be used to achieve the objects of the present invention, which publications are, for example:

Japanese Patent KOKAI (Laid-Open) Nos. 7-10839, 8-208602, 9-268172, 9-291076, 9-227496, 9-221465, 9-169719, 9-169718, 9-169721, 9-169723, 9-124584, 9-52823, 9-31045, 8-319226, 8-311012, 8-259515, 8-225514, 8-99950, 8-92215, 8-12643, 8-27093, 7-304729, 7-291927, 7-224022, 7-109251, 7-76566, 7-89938, 6-345715, 6-345643, 6-256291, 6-234730, 6-256290, 6-239828, 6-228082, 6-116230, 5-339228, 8-291131, 8-41028, 7-206823, 7-82234, 7-145149, 9-124583, 9-52876, 8-311011, 8-245560, 8-269001, 8-283232, 8-73427, 8-59598, 8-59602, 8-188568, 8-27113, 7-267926, 8-225513, 9-77753, 9-59245, 9-67332, 9-67340 and 8-277269; PCT KOKAI (Laid-Open) Nos. 6-509798, 9-505035, 9-504535, 8-511243 and 9-500895; European Patent (EP) Nos. 794172, 794171, 790245, 765868 and 787728; German Patent (DE) Nos. 19548708 and 19601303, WO Nos. 9711055, 9640728, 9604241, 9725310 and 9727183.

The compounds having the $Na^+/H^+$ exchange system inhibition activity and salts thereof, which are used in the present invention, may be prepared by the methods described in the patent publications supra, etc.

The pharmaceutical composition of the present invention, useful for the treatment of ischemic brain damage, comprises as the active ingredient the compound having the $Na^+/H^+$ exchange system inhibition activity or salt thereof. These compounds having the $Na^+/H^+$ exchange system inhibition activity or salt thereof may freely take optional forms in the pharmaceutical composition without any restriction. This compound or salt thereof may be formulated into a pharmaceutical preparation suitable for oral or parenteral administration. The compound or salt thereof described above may also be employed in the pharmaceutical preparation after the compound or salt is dissolved in distilled water or a buffer solution. In the case that the active compound is used in the form of injection, a solution, suspension or emulsion of the compounds is prepared, using conventional substances for this purpose, for example, a solubilizer, an emulsifier or other additives. Solvents available for the purpose of the present invention are, for example, water, physiological saline, an alcohol such as ethanol or propanol, or a sugar solution such as a glucose solution or a mannitol solution, and a mixture of the various solvents described above. The pharmaceutical composition of the present invention may further contain, if necessary and desired, other pharmaceutical additives, such as a surfactant, an emulsifier and a stabilizer. The pharmaceutical composition of the present invention contains the active compound in a concentration of, for example, approximately 0.1 to 10 wt%. The composition in a non-parenteral preparation may be prepared, for example, by mixing the active compound with appropriate carriers conventionally acceptable for this purpose, for example, an excipient, a stabilizer, a diluent, a binder, etc. and then treating the resulting mixture in a conventional manner into an appropriate dosage mode, such as tablets, coated tablets, capsules, granules, powders, syrup or suspensions. The excipient that may be used in the pharmaceutical composition of the present invention are, for example, gum arabic, magnesia, magnesium carbonate, calcium phosphate, lactose, glucose or starch, especially corn starch.

The dosage and frequency of administration may vary and may be determined preferably depending upon the age, body weight, condition and administration route. In general, the composition of the present invention is administered in a daily dose of 0.1 to 2000 mg, preferably 1 to 200 mg, per day for adult in a single dose or by multiple dose.

The present invention is described below more specifically with reference to Examples but is not deemed to be limited thereto.

Compound 1 used in Examples 1 and 4 is 1-methyl-2-indoloylguanidine methanesulfonate, which is described in Example 240 of Japanese Patent KOKAI (Laid-Open) No. 8-208602, namely, the methanesulfonate of the compound represented by the formula (1g); Compound 2 used in Examples 2 and 5 is 1,4-dimethyl-2-indoloylguanidine methanesulfonate, which is described in Example 241 of Japanese Patent KOKAI (Laid-Open) No. 8-208602, and Compound 3 used in Examples 3 and 6 is N-(aminoiminomethyl)-5,6,7,8-tetrahydro-11-chloro-8-oxo-4H-azocino[3,2,1-hi]indole-2-carboxamide methanesulfonate monohydrate, which is described in Example 22 of Japanese Patent KOKAI (Laid-Open) No. 10-237073, namely, the methanesulfonate of the compound represented by the formula (1h).

Example 1

Effect of $Na^{\pm}/H^{\pm}$ exchanger inhibitor on the infarcted area induced in transient ischemic rats

Male rats were anesthetized with halothane mixed with nitrous oxide. The cervical region was dissected to ligate the left external carotid artery and the left common carotid artery. Occlusion was induced for 2 hours in the control section at the origin of the middle cerebral artery by inserting a nylon thread through the left internal carotid artery to reach the left middle deutocerebral artery. Reperfusion through the middle cerebral artery was established by withdrawing the nylon thread. The test compound was intravenously given to the animals in a volume of 1 ml/kg at 30 minutes after the occlusion. As the test compound, a solution of Compound 1 (1 mg/ml) in 8% polyethylene glycol #400 was used. For control, the vehicle (8% polyethylene glycol #400) containing no active compound was used. The animals were decapitated 22 hours after reperfusion and the brain was removed. Coronal sections of 2 mm thick were prepared

in the ipsilateral hemisphere from 3 mm anterior to 5 mm posterior of the bregma. The sections were stained with 2,3,5-triphenyltetrazolium chloride for photographing. The photographs were scanned with a scanner (GT-8000, Epson) for computer-based image analysis (software used, Ep Scan Mac 1. 40). The rate of the infarcted area of each section in the left hemisphere was analyzed by a computer (software used, NIH image 1.59). The pharmacological effect was evaluated by mean percentage of the infarcted areas in the five sections obtained in one rat.

By the left middle cerebral artery occlusion for 2 hours, mean infarcted area of 47.9 ± 3.6% in each section (mean ± standard error in 9 cases) was observed 22 hours after reperfusion in the control group. In the group treated with Compound 1, mean infarcted area observed in each section was only 35.4 ± 3.1% (mean ± standard error in 8 cases, p<5%, Student's t-test). The data reveals that the infarcted area was significantly reduced in the Compound 1-treated group, as compared to the control group. The infarcted area of each section in the left hemisphere is expressed by mean ± standard error, which is shown in Fig. 1.

Example 2

Effect of $Na^{\pm}/H^{\pm}$ exchanger inhibitor on the infarcted area induced in transient ischemic rats

Compound 2 was elucidated for the effect on the infarcted area in a manner similar to that of Example 1. The test compound was intraperitoneally given 30 minutes before the occlusion to the animals in a volume of 1 ml/kg. As the test compound, a solution of Compound 2 (1 mg/ml) in 8% polyethylene glycol #400 was used. For control, the vehicle (8% polyethylene glycol #400) containing no active compound was used.

In the control group, the mean percentage of the infarcted area in each section was 49.5 ± 4.1% (mean ± standard error in 6 cases). In the drug-treated group, the infarcted area observed in each section was only 26.6 ± 3.1% (mean ± standard error in 5 cases). The data reveals that the infarcted area was significantly reduced in the drug-treated group with p<1% (by Student's t-test), as compared to the control group. The infarcted area of each section in the left hemisphere is expressed by mean percentage with standard error, which is shown in Fig. 2.

Example 3

Effect of $Na^{\pm}/H^{\pm}$ exchanger inhibitor on the infarcted area induced in transient ischemic rats

Compound 3 was elucidated for the effect on the infarcted area in a manner similar to that of Example 1. The test compound was intraperitoneally given immediately after the occlusion to the animals in a volume of 1 ml/kg. As the test compound, a solution of Compound 3 (1 mg/ml) in 8% polyethylene glycol #400 was used. For control, the vehicle (8% polyethylene glycol #400) containing no active compound was used.

In the control group, the mean percentage of the infarcted area in each section was 55.4 ± 2.1% (mean ± standard error in 14 cases). In the drug-treated group, the infarcted area observed in each section was only 45.2 ± 2.2% (mean ± standard error in 13 cases). The data reveals that the infarcted area was significantly reduced in the drug-treated group with p<5% (by Student's t-test), as compared to the control group. The infarcted area of each section in the left hemisphere is expressed by mean percentage with standard error, which is shown in Fig. 3.

Example 4

Effect of $Na^{\pm}/H^{\pm}$ exchanger inhibitor on brain edema induced in rats with transient ischemia

Male rats were anesthetized with halothane mixed with nitrous oxide. The cervical region was dissected to ligate the left external carotid artery and the left common carotid artery. Occlusion was induced for 2 hours in the control section at the origin of the middle cerebral artery by inserting a nylon thread through the left internal carotid artery to reach the left middle deutocerebral artery. Reperfusion through the middle cerebral artery was established by withdrawing the nylon thread. The test compound was intravenously given to the animals an hour after the occlusion in a volume of 1 ml/kg. As the test compound, a solution of Compound 1 (0.3 mg/ml or 1 mg/ml) in 8% polyethylene glycol #400 was used. For control, the vehicle alone (8% polyethylene glycol #400) containing no active compound was used. The animals were decapitated 4 hours after reperfusion and the brain was removed. Coronal sections of 4 mm thick were prepared in the ipsilateral hemisphere from 1 mm anterior to 3 mm posterior of the bregma. After the wet weight (W) of each section was weighed, the section was dried in an oven at 110°C for 24 hours and then its dry weight (D) was weighed. The brain water content (WC) was calculated according to the following equation.

$$WC = (W - D) / W \times 100$$

In the normal control group, the brain water content was 79.9 ± 0.1% (mean ± standard error in 15 cases); however, by reperfusion following the occlusion, the brain water content in the control group increased to 83.3 ± 0.3% (mean + standard error in 15 cases). The drug-treated groups showed the brain water content of 82.6 ± 0.3% and 82.2 ± 0.3% in 0.3 mg/kg and 1 mg/kg, respectively (mean ± standard error both in 16 cases). The results indicate that the drug-treated groups significantly prevented the increase in water content with p<1%, when compared to the control group (by Williams' multiple comparison). The results of mean brain water content with standard error obtained in the respective groups are shown in Fig. 4.

Example 5

Effect of $Na^{\pm}/H^{\pm}$ exchanger inhibitor on brain edema induced in rats with transient ischemia

Compound 2 was elucidated for the effect on brain edema in a manner similar to that of Example 4. The test compound was intravenously given immediately after the reperfusion to the animals in a volume of 1 ml/kg. As the test compound, a solution of Compound 2 (1 mg/ml) in 8% polyethylene glycol #400 was used. For control, the vehicle (8% polyethylene glycol #400) containing no active compound was used.

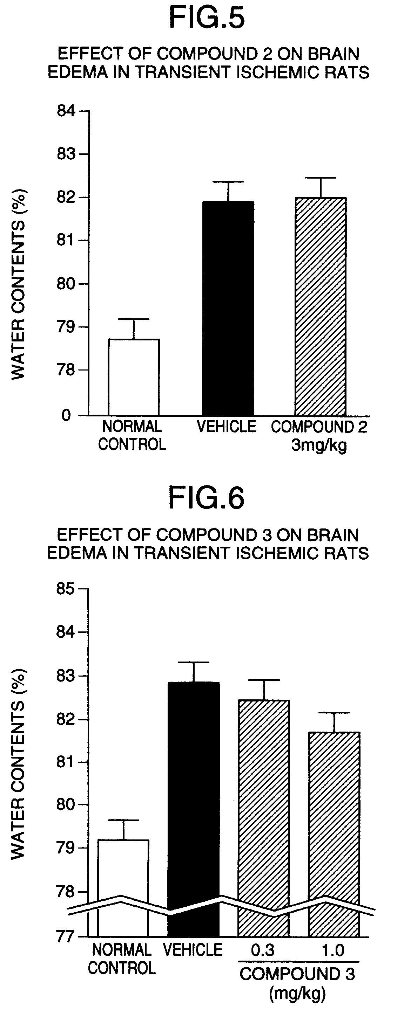

In the normal control group, the brain water content was found to be 78.7 ± 0.1% (mean ± standard error in 8 cases). By reperfusion following the ischemia, the brain water content in the control group increased to 81.9 ± 0.4% (mean ± standard error in 11 cases). In the drug-treated group, the brain water content was found to be 82.0 ± 0.4% (mean ± standard error in 11 cases); no significant effect of preventing the increase in brain water content was noted. The results of mean brain water content with standard error in each section are shown in Fig. 5.

Example 6

Effect of $Na^{\pm}/H^{\pm}$ exchanger inhibitor an brain edema induced in rats with transient ischemia

Compound 3 was elucidated for the effect on brain edema in a manner similar to that of Example 4. The test compound was intravenously given immediately after the reperfusion to the animals in a volume of 1 ml/kg. As the test compound, a solution of Compound 3 (0.3 mg/ml or 1 mg/ml) in 8% polyethylene glycol #400 was used. For control, the vehicle alone (8% polyethylene glycol #400) containing no active compound was used.

In the normal control group, the brain water content was found to be 79.2 ± 0.2% (mean ± standard error in 7 cases). By reperfusion following the ischemia, the brain water content in the control group increased to 82.8 ± 0.3% (mean ± standard error in 10 cases). In the drug-treated groups, the brain water content was found to be 82.4 ± 0.3% and 81.7 ± 0.4%, respectively, in 0.3 mg/kg and 1 mg/kg (mean ± standard error in 9 cases and 11 cases, respectively). The data reveal the tendency that the drug-treated groups prevented the increase in brain water content, as compared to the control group. The results of mean brain water content with standard error in each section are shown in Fig. 6.

It is established by these experiments in the Examples above that the $Na^+/H^+$ exchanger inhibitors exhibit good effects on ischemia-induced brain edema and cerebral infarction and therefore, effective for the treatment of ischemic brain damage.

The present invention thus provides the pharmaceutical compositions comprising the $Na^+/H^+$ exchanger inhibitors which are effective for the treatment of ischemic brain damage.

**Claims**

1. Use of a compound which has $Na^+/H^+$ exchange system inhibition activity, or a pharmaceutically acceptable salt thereof, in the manufacture of a pharmaceutical composition for the treatment of ischemic brain damage.

2. Use according to claim 1 wherein the compound is a substituted guanidine derivative, or a pharmaceutical acceptable acid addition salt thereof, which has $Na^+/H^+$ exchange system inhibition activity.

3. Use according to claim 1 or 2 wherein the compound is selected from the following compounds (a), (b), (c), (d), (e) and (f):

Compound (a) is an indoloyl guanidine derivative of formula (1):

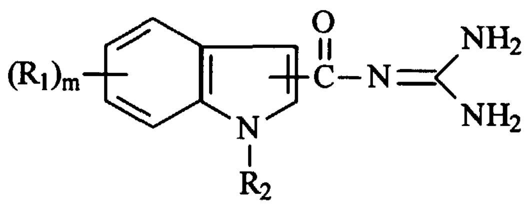

(1)

in which:

m is 1, 2, 3, 4 or 5;
each $R_1$, which may be the same or different, is hydrogen, $C_1$-$C_8$ alkyl, $C_2$-$C_6$ alkenyl, $C_2$-$C_6$ alkynyl, $C_3$-$C_7$ cycloalkyl, a halogen, nitro, $C_2$-$C_8$ alkanoyl, arylalkanoyl having up to 10 carbon atoms, aroyl having up to 11 carbon atoms, carboxyl, $C_2$-$C_6$ alkoxycarbonyl, an aromatic group, a group of formula: $-OR_3$, $-NR_6R_7$, $-SO_2NR_6R_7$ or $-S(O)_nR_{40}$, or a group of formula:

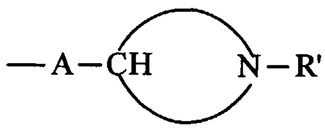

wherein A is oxygen or a group of formula: $-S(O)_n-$ or $-N(R_{50})-$ wherein $R_{50}$ is hydrogen or $C_1$-$C_8$ alkyl, R' is hydrogen, $C_1$-$C_8$ alkyl or substituted $C_1$-$C_8$ alkyl; and the ring is a saturated $C_1$-$C_8$ alkyl; and the ring is a saturated 3 to 8-membered hereto ring containing one nitrogen atom;
$R_2$ is hydrogen, $C_1$-$C_8$ alkyl, substituted $C_1$-$C_8$ alkyl, $C_3$-$C_7$ cycloalkyl, hydroxy, $C_1$-$C_6$ alkoxy, an aromatic group or a group of formula: $-CH_2R_{20}$;
$R_3$ is hydrogen, $C_1$-$C_8$ alkyl, substituted $C_1$-$C_8$ alkyl, $C_3$-$C_7$ cycloalkyl, an aromatic group or a group of formula: $-CR_2R_{30}$, in which $R_{30}$ is $C_2$-$C_6$ alkenyl or $C_2$-$C_6$ alkynyl;
each of $R_6$ and $R_7$, which are the same or different, is independently hydrogen, $C_1$-$C_8$ alkyl, substituted $C_1$-$C_8$ alkyl, $C_3$-$C_7$ cycloalkyl, $C_2$-$C_8$ alkanoyl, arylalkanoyl having up to 10 carbon atoms, aroyl having up to 11 carbon atoms, an aromatic group or a group of formula $-CH_2R_{60}$ wherein $R_{60}$ is $C_2$-$C_6$ alkenyl or $C_2$-$C_6$ alkynyl; or $R_6$ and $R_7$ form, together with the N atom to which they are attached, a saturated 5- to 7-membered cyclic amino group which may contain other hetero atom(s) in the ring thereof;
$R_{40}$ is $C_1$-$C_8$ alkyl, substituted $C_1$-$C_8$ alkyl or an aromatic group;
n is 0, 1 or 2; and
$R_{20}$ is $C_2$-$C_6$ alkenyl or $C_2$-$C_6$ alkynyl; and wherein:
a substituted $C_1$-$C_8$ alkyl group as defined above is $C_1$-$C_8$ alkyl substituted by halogen, hydroxy, $C_1$-$C_6$ alkoxy, cyano, carboxyl, $C_2$-$C_6$ alkoxycarbonyl, $C_2$-$C_8$ alkanoyl, arylalkanoyl having up to 10 carbon atoms, aroyl having up to 11 carbon atoms, an aromatic group or $-CONR_4R_5$ in which each of $R_4$ and $R_5$, which are the same or different, is independently hydrogen or $C_1$-$C_8$ alkyl, or $R_4$ and $R_5$ form, together with the N atom to which they are attached, a saturated 5- to 7-membered cyclic amino group which may contain other hetero atom(s) in the ring; $-NR_6R_7$; or a group of formula:

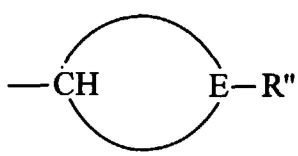

in which:
E is nitrogen or CH, and,

R" is hydrogen, $C_1$-$C_8$ alkyl or $C_1$-$C_8$ alkyl substituted by hydroxy, $C_1$-$C_6$ alkoxy, cyano, carboxyl, $C_2$-$C_6$ alkoxycarbonyl, $C_2$-$C_8$ alkanoyl, arylalkanoyl having up to 10 carbon atoms, aroyl having up to 11 carbon atoms, an aromatic group, a group of formula $-NR_6R_7$, or a group of formula $CONR_4R_5$ in which each of $R_4$ and $R_5$, which are the same or different, is independently hydrogen or $C_1$-$C_8$ alkyl, or $R_4$ and $R_5$ form together with the N atom to which they are attached a saturated 5- to 7-membered cyclic amino group which may contain other hetero atom(s) therein; and the ring of formula

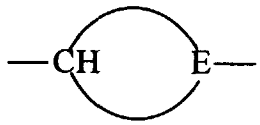

is a 3- to 8-membered saturated aliphatic ring or saturated hereto ring containing one nitrogen atom;

an aromatic group as defined above is an aryl group having up to 10 carbon atoms, a 5- or 6-membered heteroaryl group containing 1 to 4 nitrogen atom(s), a 5- or 6-membered hetero-aryl group containing 1 to 2 nitrogen atom(s) and one oxygen atom or one sulfur atom, or furyl, the said aromatic said group being unsubstituted or substituted by $C_1$-$C_8$ alkyl, substituted $C_1$-$C_8$ alkyl as defined above, halogen, nitro, $C_2$-$C_6$ alkoxycarbonyl, carboxyl or a group of formula: $-OR_3$, $-NR_6R_7$, $-CONR_6R_7$, $-SO_2NR_6R_7$ or $-S(O)_nR_{40}$ wherein $R_3$, $R_6$, $R_7$, n and $R_{40}$ are as defined above;

and wherein each $R_1$ and the guanidinocarbonyl moiety may occupy any position on the 5- or 6-membered ring of the indole nucleus;

or a pharmaceutically acceptable acid addition salt thereof;

Compound (b)is a substituted guanidine derivative of formula (1b):

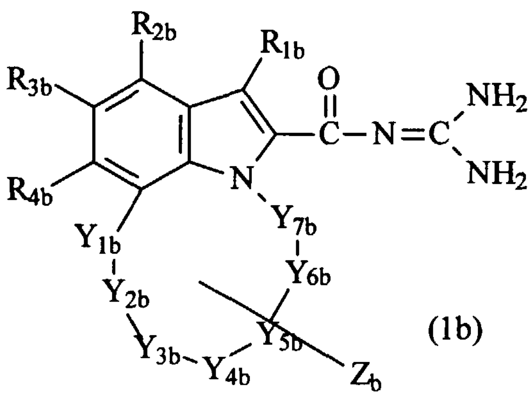

(1b)

in which:

each of $R_{1b}$, $R_{2b}$, $R_{3b}$ and $R_{4b}$, which are the same or different, is hydrogen, alkyl, cycloalkyl, cycloalkenyl, a heterocyclic or aromatic group, each said group being unsubstituted or substituted, or is halogen, nitro, carboxyl, alkoxycarbonyl, acyl, $-OR_{5b}$, $-N(R_{6b})R_{7b}$, $-CON(R_{6b})R_{7b}$, $-SO_2N(R_{6b})R_{7b}$ or $-S(O)_nR_{8b}$ wherein $R_{8b}$ is alkyl or an aromatic group as defined above and n is 0, 1 or 2, $-Q_b-R_{ab}$, or

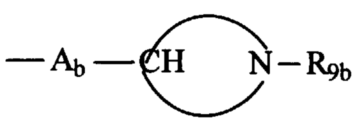

wherein $A_b$ is an oxygen atom or $-S(O)_n-$ wherein n is as defined above or $-N(R_{10b})-$, $R_{9b}$ is hydrogen,

unsubstituted or substituted alkyl, acyl or $-Q_b-R_{ab}-$, and the ring is a 3- to 8-membered saturated heterocyclic group composed of a nitrogen atom and carbon atoms;

each of $Y_{1b}$, $Y_{2b}$, $Y_{3b}$, $Y_{4b}$, $Y_{5b}$, $Y_{6b}$ and $Y_{7b}$, which are the same or different is independently a single bond, $-CH_2-$, -O-, -CO-, $-C(=C(R_{11b})R_{12b})-$, $-S(O)_n-$ or $-N(R_{10b})-$, or adjacent members of $Y_{1b}$ to $Y_{7b}$ represent together -CH=CH-, and at least two of $Y_{1b}$ to $Y_{7b}$ being independently a group other than a single bond;

$Z_b$ may be absent or one or more $Z_b$s, which may be the same or different, may be bonded to one or more of the carbon atoms constituting the ring formed by $Y_{1b}$ to $Y_{7b}$, the or each $Z_b$ being unsubstituted or substituted alkyl, alkenyl, alkynyl, cycloalkyl, cycloalkenyl, substituted heterocyclic, a halogen, carboxyl, alkoxycarbonyl, an aromatic group, acyl, $-OR_{5b}$, $-N(R_{6b})R_{7b}$, $-S(O)_nR_{8b}$, $-C(O)N(R_{6b})R_{7b}$, or $-Q_b-R_{ab}$, provided that when $Z_b$ replaces a hydrogen atom in -CH=CH- $Z_b$ is not $-N(R_{6b})R_{7b}$ or $-S(O)_nR_{8b}$;

$Q_b$ is substituted or unsubstituted lower alkylene;

$R_{ab}$ is vinyl or ethynyl, both of which are substituted or unsubstituted;

$R_{5b}$ is hydrogen, unsubstituted alkyl, substituted alkyl, cycloalkyl, cycloalkenyl, a saturated heterocyclic group or an aromatic group;

$R_{6b}$ and $R_{7b}$, which are the same or different, are each hydrogen, unsubstituted alkyl, substituted alkyl, cycloalkyl, cycloalkenyl, saturated heterocyclic, an aromatic group, acyl or $-Q_bR_{ab}$, or $R_{6b}$ and $R_{7b}$, when taken together with the nitrogen atom to which they are bonded, form a saturated 5- to 7-membered cyclic amino group which may contain an oxygen atom or a sulfur atom in the ring and may be substituted by one or more unsubstituted or substituted alkyl, hydroxyl or $-OR_{5b}$ groups;

$R_{8b}$ is an unsubstituted or substituted alkyl or an aromatic group;

$R_{10b}$ is hydrogen, unsubstituted or substituted alkyl, cycloalkyl, saturated heterocyclic, an aromatic group, acyl or $Q_b-R_{ab}$; and;

$R_{11}$ and $R_{12b}$, which are the same or different, are each hydrogen, unsubstituted or substituted alkyl, alkenyl, alkynyl, cycloalkyl, cycloalkenyl, saturated heterocyclic, a halogen atom, carboxyl, alkoxycarbonyl, an aromatic group, acyl, $-OR_{5b}$, $-N(R_{6b})R_{7b}$,- $C(O)N(R_{6b})R_{7b}$, $-S(O)_nR_{8b}$ or $-Q_b-R_{ab}$; and n is 0, 1 or 2; or a pharmaceutically acceptable acid addition salt thereof;

Compound (c) is substituted guanidine derivative of formula (1c):

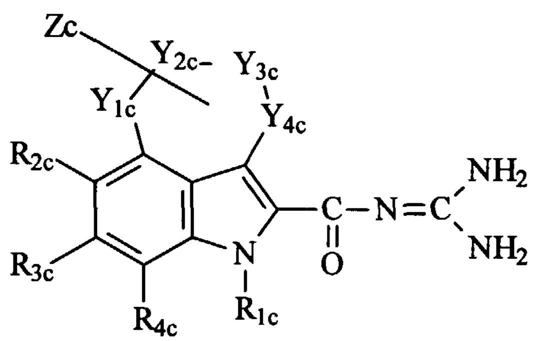

(1c)

in which:

$R_{1c}$ is hydrogen, unsubstituted or substituted alkyl, cycloalkyl, cycloalkenyl, saturated heterocyclic, an aromatic group, $-OR_{5c}$, acyl or $-Q_c-R_{ac}$;

$R_{2c}$, $R_{3c}$ and $R_{4c}$, which are the same or different, are independently hydrogen, unsubstituted or substituted alkyl, cycloalkyl, cycloalkenyl, saturated heterocyclic, a halogen, nitro, carboxyl, alkoxycarbonyl, an aromatic group, $-OR_{5c}$, $-N(R_{6c})R_{7c}$, $-CON(R_{6c})R_{7c}$, - $SO_2N(R_{6c})R_{7c}$, $-S(O)_nR_{8c}$ wherein $R_{8c}$ is unsubstituted or substituted alkyl or an aromatic group, and n is 0, 1 or 2, acyl, $-Q_c-R_{ac}$, or

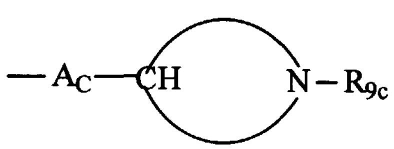

wherein the ring is a 3- to 8- membered saturated heterocyclic group composed of a nitrogen atom and carbon atoms;

$Y_{1c}$, $Y_{2c}$, $Y_{3c}$ and $Y_{4c}$ are defined as follows:

(1) one of $Y_{1c}$,$Y_{2c}$,$Y_{3c}$ and $Y_{4c}$ is methylene, carbonyl, an oxygen atom, $-S(O)_n-$, $-N(R_{10c})-$ or $-C(=C(R_{11c})(R_{12c}))-$, two others are independently a methylene group, and the remaining one is a single bond or a methylene group, or

(2) any adjacent two members of $Y_{1c}$,$Y_{2c}$,$Y_{3c}$ and $Y_{4c}$ together form a vinylene group (-CH=CH-) or $-CON(R_{10c})-$, another is methylene, carbonyl, an oxygen atom, $-S(O)_n-$, $-N(R_{10c})-$ or $-C(=C(R_{11c})(R_{12c}))-$, and the remaining one is a single bond or a methylene group, provided that the oxygen atom, nitrogen atom and sulfur atom are not directly bonded to the vinylene group;

$Z_c$ may be absent or one or more $Z_c$s, which may be the same or different, may be bonded to one or more of the carbon atoms constituting the ring formed by $Y_{1c}$ to $Y_{4c}$, the or each $Z_c$ being unsubstituted or substituted alkyl, alkenyl, alkynyl, cycloalkyl, cycloalkenyl, saturated heterocyclic, halogen, carboxyl, alkoxycarbonyl, an aromatic group, $-OR_{5c}$, $-N(R_{6c})R_{7c}$, $-CON(R_{6c})R_{7c}$, $-S(O)_nR_{8c}$, acyl, or $-Q_c-R_{ac}-$;

$A_c$ is an oxygen atom, $-S(O)_n-$ wherein n is as defined above or $-N(R_{10c})-$;

$Q_c$ is substituted or unsubstituted lower alkylene;

$R_{ac}$ is vinyl or ethynyl,both of which are substituted or unsubstituted;

$R_{5c}$ is hydrogen, unsubstituted or substituted alkyl, cycloalkyl, cycloalkenyl, saturated heterocyclic or an aromatic group;

$R_{6c}$ and $R_{7c}$, which may be the same or different, are independently hydrogen, unsubstituted or substituted alkyl, cycloalkyl, cycloalkenyl, saturated heterocyclic, an aromatic group, acyl or $-Q_c-R_{ac}$, or $R_{6c}$ and $R_{7c}$, when taken together with the nitrogen atom to which they are bonded, form a saturated 5- to 7-membered cyclic amino group which may contain an oxygen atom or a sulfur atom in the ring and may be substituted by one or more unsubstituted or substituted alkyl, hydroxyl or $-OR_{5c}$ groups;

$R_{8c}$ is unsubstituted or substituted alkyl or an aromatic group;

$R_{9c}$ is hydrogen, unsubstituted or substituted alkyl, acyl or $-Q_c-R_{ac}$,

$R_{10}$ is hydrogen, unsubstituted or substituted alkyl, cycloalkyl, saturated heterocyclic, an aromatic group, acyl or $-Q_c-R_{ac}$; and

$R_{11c}$ and $R_{12c}$, which may be the same or different, are independently hydrogen, unsubstituted or substituted alkyl, alkenyl, alkynyl, cycloalkyl, cycloalkenyl, saturated heterocyclic, a halogen, carboxyl, alkoxycarbonyl, an aromatic group, $-OR_{5c}$, $-N(R_{6c})R_{7c}$, $-CON(R_{6c})R_{7c}$, $-S(O)_nR_{8c}$, acyl or $-Q_c-R_{ac}-$; and n is 0, 1 or 2;

or a pharmaceutically acceptable acid addition salt thereof;

Compound (d) is a substituted guanidine derivative of formula:

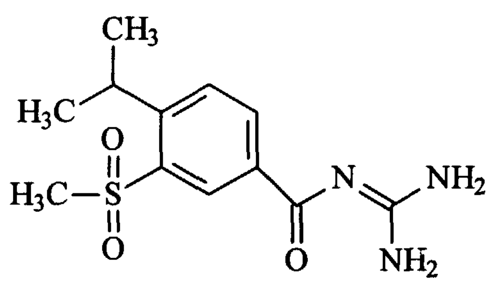

(1d)

or a pharmaceutically acceptable acid addition salt thereof;

Compound (e) is a substituted guanidine derivative of formula (1e):

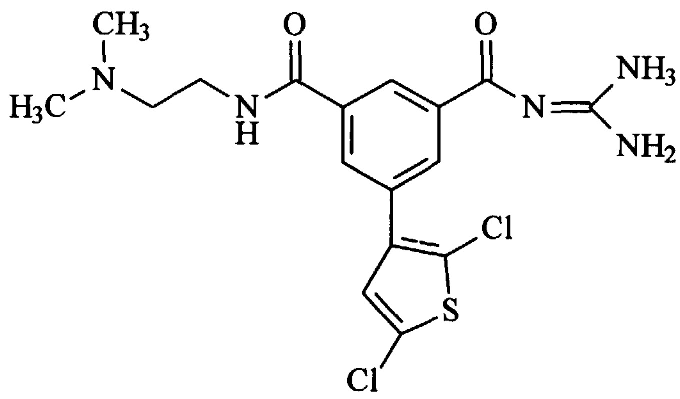

(1e)

or a pharmaceutically acceptable acid addition salt thereof; and,
Compound (f) is a substituted guanidine derivative of formula (1f):

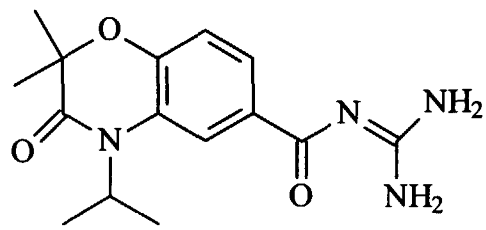

(1f)

or a pharmaceutically acceptable acid addition salt thereof.

**4.** Use according to claim 3, wherein the compound is Compound (a).

**5.** Use according to claim 3, wherein the compound is Compound (b).

**6.** Use according to claim 3, wherein the compound is selected from 1-methyl-2-indoloylguanidine, N-(aminoiminomethyl)-5,6,7,8-tetrahydro-11-chloro-8-oxo-4H-azocino[3,2,1-hi]indole-2-carboxamide, 1-methyl-2-indoloylguanidine and N-(aminoiminomethyl)-5,6,7,8-tetrahydro-11-chloro-8-oxo-4H-azocino[3,2,1-hi]indole-2-carboxamide, and the pharmaceutically acceptable acid addition salts thereof.

**7.** Use according to any one of claim 1 to 6 wherein the ischemic brain damage is cerebral infarction.

**8.** Use according to any one of claims 1 to 6 wherein the ischemic brain damage is cerebral embolism.

**9.** Use according to any one of claims 1 to 6, wherein the ischemic brain damage is cerebral thrombus.

FIG.1

EFFECT OF COMPOUND 1 ON INFARCTED AREA IN TRANSIENT ISCHEMIC RATS

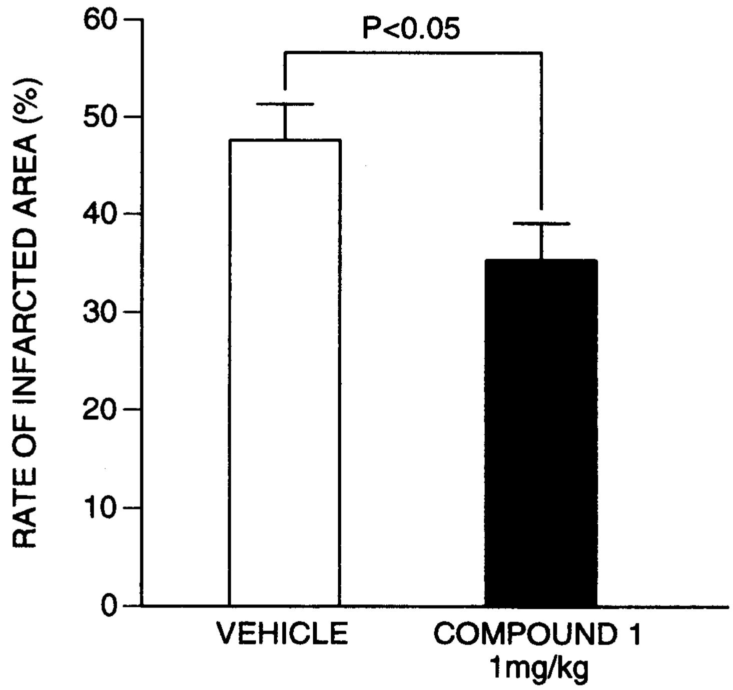


FIG.2

EFFECT OF COMPOUND 2 ON INFARCTED AREA IN TRANSIENT ISCHEMIC RATS

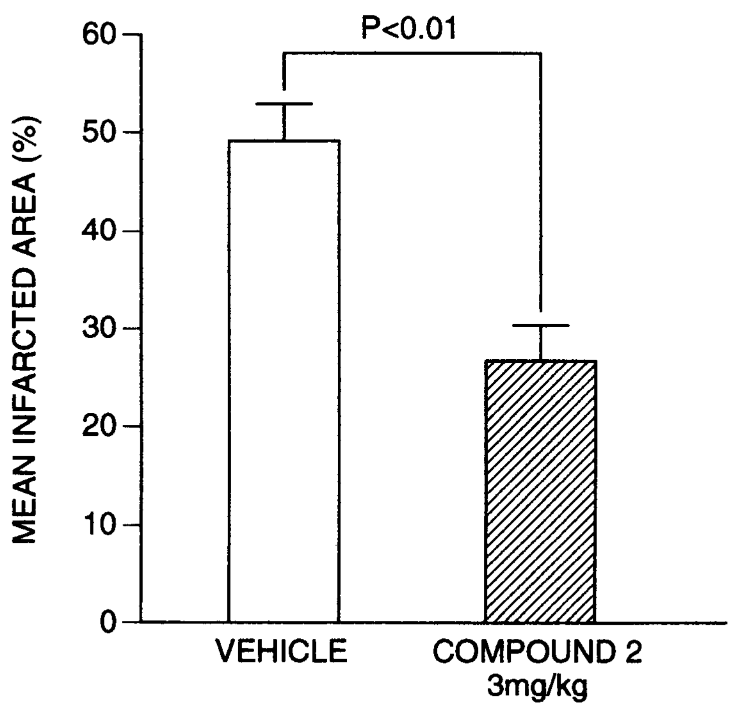

# FIG.3

EFFECT OF COMPOUND 3 ON INFARCTED AREA IN TRANSIENT ISCHEMIC RATS

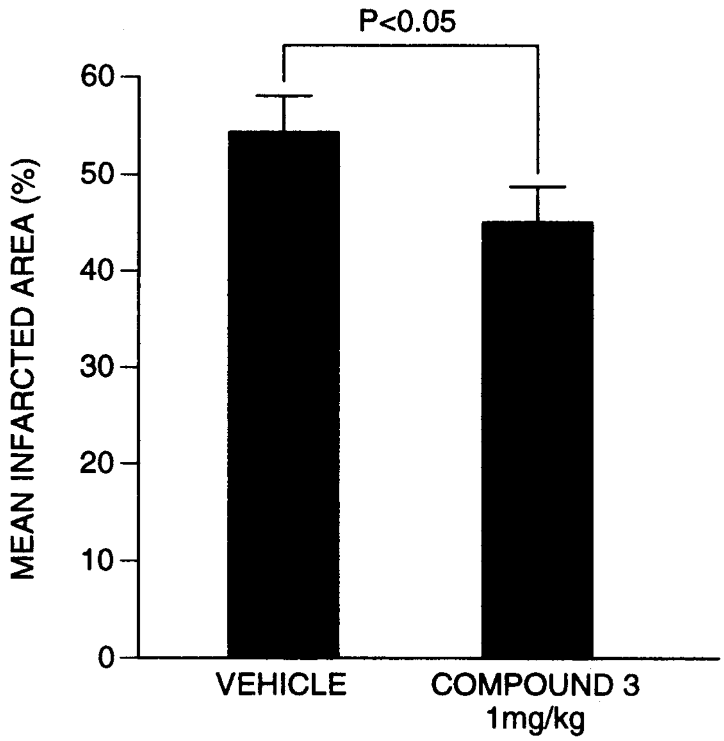


# FIG.4

EFFECT OF COMPOUND 1 ON BRAIN EDEMA IN TRANSIENT ISCHEMIC RATS

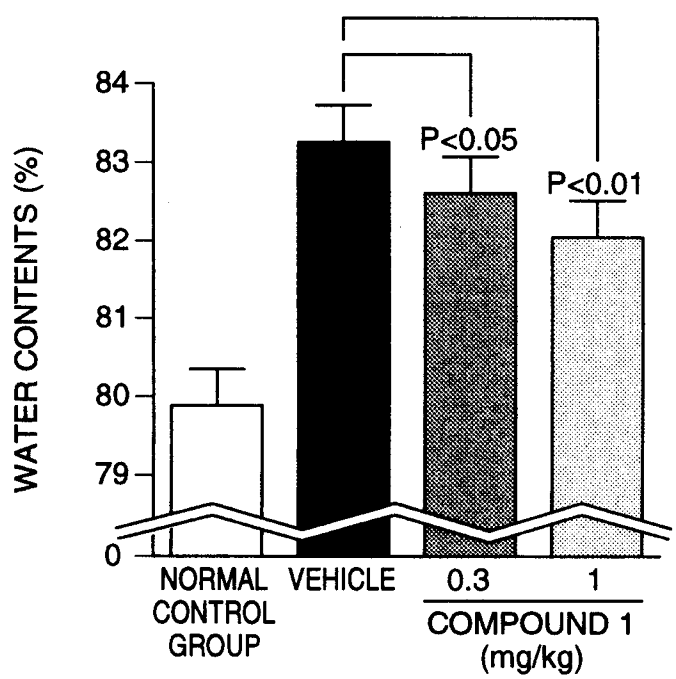

# FIG.5

EFFECT OF COMPOUND 2 ON BRAIN EDEMA IN TRANSIENT ISCHEMIC RATS

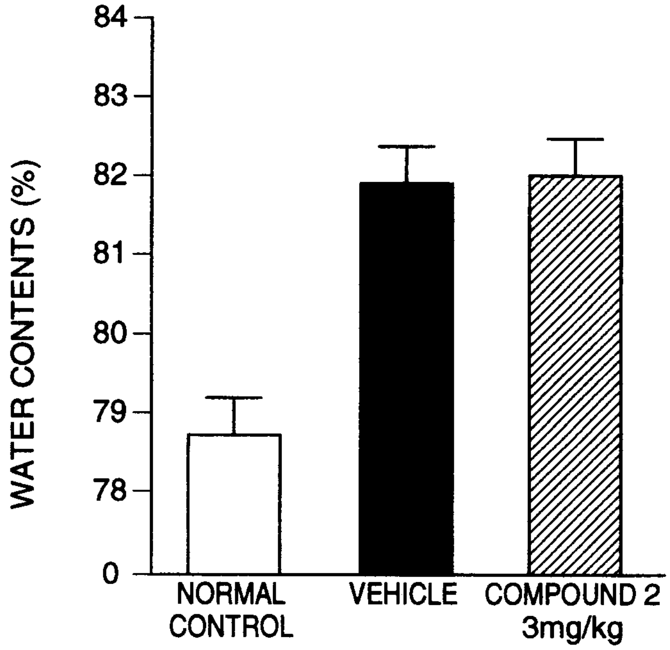


# FIG.6

EFFECT OF COMPOUND 3 ON BRAIN EDEMA IN TRANSIENT ISCHEMIC RATS